# EUROPEAN PATENT APPLICATION

(11) **EP 1 884 230 A1**
(43) Date of publication of application: **06.02.2008**
(21) Application number: 06746125.1
(22) Date of filing: 09.05.2006
(51) Int. Cl.: A61K 8/44, A61K 8/46, A61Q 19/00

(54) **PARAKERATOSIS INHIBITOR, PORE-SHRINKING AGENT OR AGENT FOR PREVENTING/AMELIORATING ROUGH SKIN AND EXTERNAL COMPOSITION FOR SKIN**

(30) Priority: 25.05.2005 JP 2005151983
(71) Applicant: Shiseido Company, Limited, Chuo-ku Tokyo 104-8010 (JP)
(72) Inventor: KAMINUMA, Mikiko c/o SHISEIDO RESEARCH CENTER, Yokohama-shi, Kanagawa 2248558 (JP); SUETSUGU, Masaru c/o SHISEIDO RESEARCH CENTER, Yokohama-shi, Kanagawa 2248558 (JP); KANEKO, Maki c/o SHISEIDO RESEARCH CENTER, Yokohama-shi, Kanagawa 2248558 (JP); IIDA, Toshii c/o SHISEIDO RESEARCH CENTER, Yokohama-shi, Kanagawa 2248558 (JP); INOMATA, Shinji c/o SHISEIDO RESEARCH CENTER, Yokohama-shi, Kanagawa 2248558 (JP)
(74) Representative: Merkle, Gebhard
(86) International application number: PCT/JP2006/309294
(87) International publication number: WO 2006/126385

(57) **Abstract**

The invention provides a parakeratosis inhibitor, pore-shrinking agent, or rough skin preventing/amaliorating agent that has a function such as parakeratosis inhibition, pore shrinkage, or rough skin -inhibition/abatement, poses no safety problems such as sensory irritation, and is very safe, and to further provide an external composition for skin to which a compound having the above-mentioned function has been added. The parakeratosis inhibitor agent, pore-shrinking agent, or rough skin preventing/amaliorating agent comprises one, two, or more compounds selected from the group consisting of α-amino acid derivatives and salts thereof. The external composition for skin comprises the one, two, or more compounds selected from the group consisting of α-amino acid derivatives and salts thereof as the above-mentioned parakeratosis inhibitor, pore-shrinking agent, or rough skin preventing/amaliorating agent.

## Description

### TECHNICAL FIELD

The present invention relates to a parakeratosis inhibitor for inhibiting parakeratosis caused by sebum; a pore-shrinking agent for inhibiting parakeratosis caused by the irritating components in the sebum around the pore, maintaining normal skin conditions around the pore, and suppressing a conical structure of the pore from becoming conspicuous; a rough skin preventing/ameliorating- agent with which rough skin caused by unsaturated fatty acids is prevented/ameliorating; and an external composition for skin having a function such similarly to inhibiting parakeratosis, shrinking pores, or preventing and ameliorating rough skin.

### BACKGROUND ART

Today young women in particular are very concerned with conspicuous pores, and an external composition for skin that provides relief for this condition is necessary. However, the mechanism by which pores become conspicuous has not been elucidated, and conventional treatment has been through the use of an astringent cosmetics toner or the removal of keratin plug. Alternatively, a foundation is often used in order to improve appearance. However, the purpose of an astringent cosmetics toner is to tighten the skin, to temporarily reduce the skin surface temperature by an alcohol, or to coagulate the proteins by an organic acid and the like. Consequently, the skin is temporarily tightened, and the results have therefore been unsatisfactory in that there is an increase in stress applied to the skin without a fundamental solution to conspicuous pores.

On the other hand, although there are reports of the pore-shrinking effect derivatives of glycolic acid and ascorbic acid (for example, see Non-Patent Document 1), there are still many unknowns , such as the mechanism of action, the extent of the effect, and the like.

Keratin plug removal is the physical removal of a keratin plug clogging the pore. Conventional removal methods include agents for keratin plug removal containing polymer compounds having salt-generating groups (for example, see Patent Document 1); cosmetics containing water-insoluble cyclodextrin polymers (for example, see Patent Document 2); and cosmetics for keratin plug removal containing 50 mass% or greater of an oil component having a viscosity of 5 to 80 mPa·s/25°C (for example, see Patent Document 3). The physical force of such methods for keratin plug removal can damage the skin, and side effects on the skin has been a serious problem. The effect of this method is not always satisfactory since the effect thereof is temporary and keratin plug is readily regenerated, and removal of keratin plug may only expand the pore.

The inventors performed considerable research on the mechanism of conspicuous pores in order to develop an external composition for skin that ameliorates conspicuous pores, and discovered such features, among others, as those listed herein below and presented the same at the 102^{nd} Convention of the Japanese Dermatological Association (see Non-Patent Document 2).(1) the conical depression surrounding a follicle is recognized as a pore, and when this portion is enlarged, the pore becomes conspicuous;
(2) the stratum corneum of this conical area is in a state of parakeratosis (nuclei that should have disappeared still remain);
(3) people having conspicuous pores also have a large amount of sebum, particularly unsaturated fatty acids;
(4) these unsaturated fatty acids are the cause of parakeratosis;
(5) it is highly probable that the unsaturated fatty acids in sebum are the cause of conspicuous pores, and the like.

It was made clear from the above-mentioned observations that sebum-associated parakeratosis is one mechanism by which pores become conspicuous. It was also made clear that conspicuous pores can be abated by ameliorating the parakeratosis.

Similarly to addition, as a result of searching for drugs having the above-mentioned parakeratosis-inhibiting effect and pore-shrinking effect, it was discovered that antagonists to stimulatory cell receptors and agonists to inhibitory cell receptors have these functions (see Patent Document 4). Examples of the former antagonists may include, for example, D-glutamic acid and TNP-ATP. Examples of the latter agonists may include, for example, β-alanine, GABA, serine, and taurine.

Nevertheless, there is a need for the development of a superior compound because no conventional compound is satisfactory as a parakeratosis inhibitor, pore-shrinking agent, or rough skin preventing/ameliorating agent in that they have insufficient effect, such as parakeratosis-inhibiting effect, pore-shrinking effect, and rough skin-preventing/ameliorating effect; they are sensory irritants; and there are limits to the amount that can be added to an external composition for skin, and the like.

Patent Document 1: Japanese Laid-Open Patent Application No. 5-97627
Patent Document 2: Japanese Laid-Open Patent Application No. 5-105619
Patent Document 3: Japanese Laid-Open Patent Application No. 2002-241260
Patent Document 4: Japanese Patent Application No. 2002-153457
Non-Patent Document 1: Yazawa et al., Aroma Research, 2002, volume 30, No. 2, p. 54-58.
Non-Patent Document 2: lida et al., Program and Minutes of the 102nd Japanese Dermatological Association Convention, 2003, 103, p. 846.

### DISCLOSURE OF THE INVENTION

### PROBLEMS TO BE SOLVED BY THE INVENTION

The present invention was completed in light of the above-mentioned facts, and an object thereof is to provide a parakeratosis inhibitor, pore-shrinking agent, or rough skin preventing/ameliorating agent that has a function such as parakeratosis inhibition, pore shrinkage, or rough skin inhibition/abatement, poses no safety problems such as sensory irritation, and is high in safety, and to further provide an external composition for skin to which a compound having the above-mentioned function has been added.

### MEANS USED TO SOLVE THE ABOVE-MENTIONED PROBLEMS

In order to solve the above-mentioned problems, the inventors conducted investigation and research on the compounds having an inhibiting effect on parakeratosis caused by unsaturated fatty acid based on the above-mentioned discoveries. As a result, the inventors discovered that specific α-amino acid derivatives and salts thereof have the above-mentioned effect, are not sensory irritants, are high in safety, and solve the above-mentioned problems, whereby the inventors completed this invention.

Specifically, the present invention is a parakeratosis inhibitor, pore-shrinking agent, or rough skin preventing/ameliorating agent comprising one, two, or more compounds selected from the group consisting of an α-amino acid derivative represented by general formula (1), or a salt thereof.

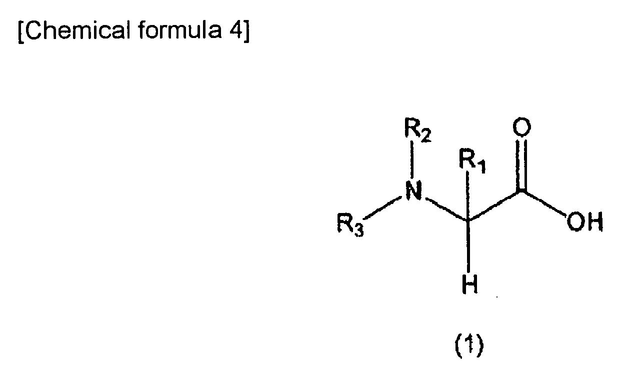

(In general formula (1), R₁ represents a hydrogen atom, a CH₃ group, or a CH₂OH group. R₂ and R₃ each independently represents a hydrogen atom, an alkyl group having 1 to 3 carbons, an allyl group, a carbobenzoyloxy group, a group represented by the following general formula (2), or a group represented by the following general formula (3). R₂ and R₃ cannot both be hydrogen groups. When R₁ is a hydrogen atom, one of R₂ and R₃ is a group represented by general formula (2) or (3).)

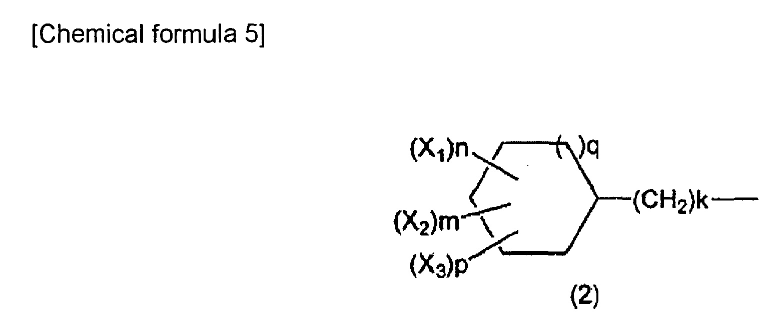

(In general formula (2), X₁, X₂, and X₃ each independently represents an alkyl group having 1 to 4 carbons, an alkoxy group having 1 to 4 carbons, a hydroxyl group, an amino group, a monoalkylamino or dialkylamino group having 1 to 4 carbons, a fluorine atom, or a trifluoromethyl group. n, m, and p each independently represents an integer of 0 to 3, and k and q each independently represents an integer of 0 to 2.)

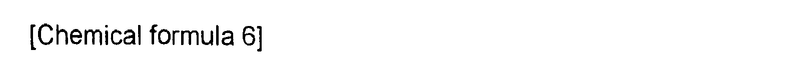

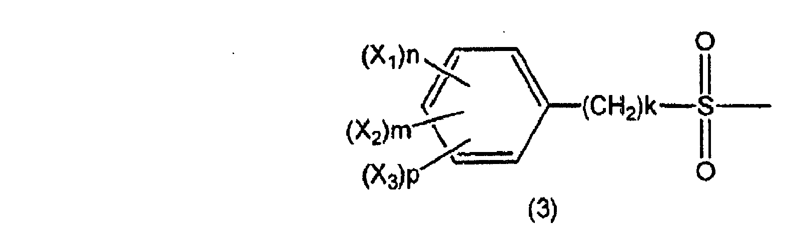

(In general formula (3), X₁, X₂, X₃, k, n, m, and p are the same similarly to general formula (2).)

Preferably one of R₂ and R₃ in general formula (1) is a hydrogen atom.

Preferably one of R₂ and R₃ in general formula (1) of claim 1 is an alkyl group having 1 to 3 carbons, and it is particularly preferred that (the other) one of R₂ and R₃ is a hydrogen atom.

Preferably one of R₂ and R₃ in general formula (1) is a carbobenzoyloxy group, and it is particularly preferred that (the other) one of R₂ and R₃ is a hydrogen atom.

Preferably one of R₂ and R₃ in general formula (1) is a cyclohexyl group, and it is particularly preferred that (the other) one of R₂ and R₃ is a hydrogen atom.

Preferably one of R₂ and R₃ in general formula (1) is a benzenesulfonyl group, and it is particularly preferred that (the other) one of R₂ and R₃ is a hydrogen atom.

The α-amino acid derivative represented by general formula (1) is preferably *N*-methyl-L-serine, *N*-methyl-DL-serine, *N*-methyl-D-serine, *N*-ethyl-L-serine, *N*-ethyl-DL-serine, *N*-ethyl-D-serine, *N*-methyl-L-alanine, *N*-methyl-DL-alanine, *N*-methyl-D-alanine, *N*-ethyl-L-alanine, *N*-ethyl-DL-alanine, *N*-ethyl-D-alanine, N-carbobenzoyloxy-L-serine, *N*-carbobenzoyloxy-DL-serine, *N*-carbobenzoyloxy-D-serine, *N-*carbobenzoyloxy-L-alanine, *N*-carbobenzoyloxy-DL-alanine, *N*-carbobenzoyloxy-D-alanine, *N*-cyclohexyl-glycine, *N*-cyclohexyl-DL-serine, *N*-cyclohexyl-L-serine, *N-*cydohexyl-D-serine, *N*-cyclohexyl-L-alanine, *N*-cyclohexyl-DL-alanine, *N*-cyclohexyl-D-alanine, N-benzenesulfonyl-glycine, *N*-benzenesulfonyl-L-serine, *N-*benzenesulfonyl-DL-serine, *N*-benzenesulfonyl-D-serine, *N*-benzenesulfonyl-L-alanine, *N*-benzenesulfonyl-DL-alanine, or *N*-benzenesulfonyl-L-alanine.

The above-mentioned parakeratosis inhibitor, pore-shrinking agent, or rough skin preventing/ameliorating agent can be prepared as an external composition for skin.

### EFFECT OF THE INVENTION

The present invention provides a very safe parakeratosis inhibitor for inhibiting parakeratosis caused by sebum; a pore-shrinking agent for inhibiting parakeratosis caused by the irritating components of sebum around pores, normalizing the skin around pores, and preventing conspicuous conical structure in pores; and a rough skin preventing/ameliorating agent with which rough skin caused by unsaturated fatty acids is prevented/abated. The present invention also provides an external composition for skin having a function such similarly to inhibiting parakeratosis, shrinking pores, or preventing/ameliorating rough skin.

### BEST MODE FOR CARRYING OUT THE INVENTION

The present invention will now be described in detail.

The present invention uses one, two, or more compounds selected from the group consisting of α-amino acid derivatives represented by the following general formula (1), and salts thereof.

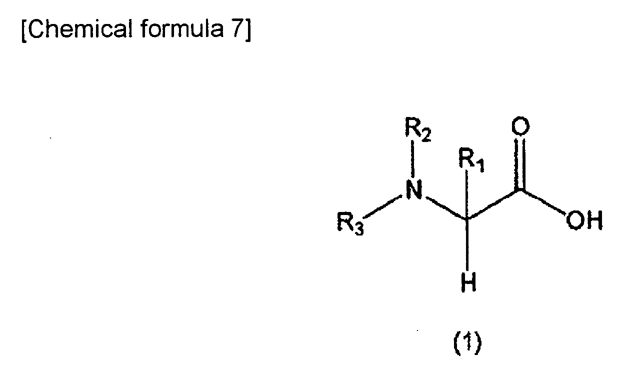

(In general formula (1), R₁ represents a hydrogen atom, a CH₃ group, or a CH₂OH group. R₂ and R₃ each independently represents a hydrogen atom, an alkyl group having 1 to 3 carbons, an allyl group, a carbobenzoyloxy group, a group represented by the following general formula (2), or a group represented by the following general formula (3). R₂ and R₃ cannot both be hydrogen groups. When R₁ is a hydrogen atom, one of R₂ and R₃ is a group represented by general formula (2) or (3).)

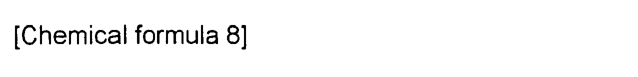

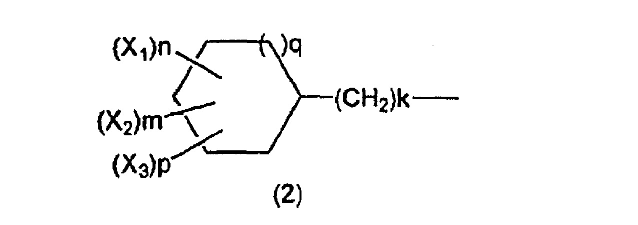

(In general formula (2), X₁, X₂, and X₃ each independently represents an alkyl group having 1 to 4 carbons, an alkoxy group having 1 to 4 carbons, a hydroxyl group, an amino group, a monoalkylamino or dialkylamino group having 1 to 4 carbons, a fluorine atom, or a trifluoromethyl group. n, m, and p each independently represents an integer of 0 to 3, and k and q each independently represents an integer of 0 to 2.)

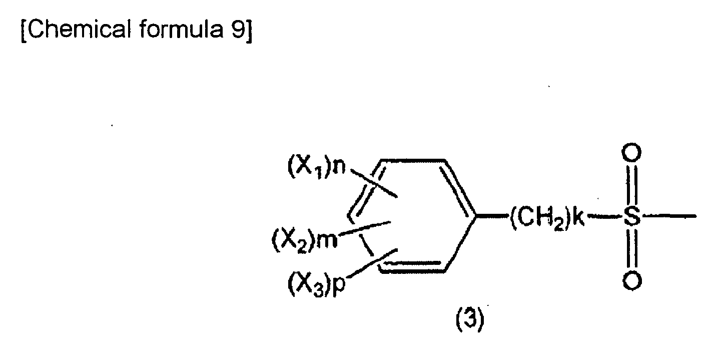

(In general formula (3), X₁, X₂, X₃, k, n, m, and p are the same similarly to general formula (2).)

The α-amino acid derivatives represented by general formula (1) will now be described.

In general formula (1), R₁ represents a hydrogen atom, CH₃ group, or CH₂OH group. The present invention is the corresponding glycine derivative when R₁ is a hydrogen atom; the corresponding alanine derivative when R₁ is a CH₃ group; and the corresponding serine derivative when R₁ is a CH₂OH group. The alanine derivative may be the D-form, L-form, or DL-form (DL mixture) because the effect is good with any form, and the DL-form (DL mixture) may have any mixture ratio. The serine derivative may be the D-form, L-form, or DL-form (DL mixture) because the effect is good with any form, and the DL-form (DL mixture) may have any mixture ratio.

R₂ and R₃ each independently represents a hydrogen atom, an alkyl group having 1 to 3 carbons, an allyl group, a carbobenzoyloxy group, or a group represented by the above-mentioned general formula (2) or (3).

R₂ and R₃ cannot both be hydrogen groups. With the exception of cases where R₁ is a hydrogen atom (glycine derivative), R₂ and R₃ can form any combination as long as the groups remain within the indicated scope. When R₁ is a hydrogen atom, one of R₂ and R₃ is a group represented by general formula (2) or (3). In this case, as long as one group is a group represented by one of these formulas, (the other) group can be any group within the scope of the present invention.

Preferably one of R₂ and R₃ of the α-amino acid derivatives of the present invention is a hydrogen atom, an alkyl group having 1 to 3 carbons, or allyl group because the effect of the present invention will be good. A hydrogen atom is most preferred because the effect will be better.

Specific examples of an alkyl groups having 1 to 3 carbons may include, for example, methyl, ethyl, *n*-propyl, and *iso*-propyl groups. A methyl or ethyl group is preferred because the effect of the α-amino acid derivatives of the present invention will be good.

The effect will be good when one of R₂ and R₃ is an alkyl group having 1 to 3 carbons as long as (the other) one is within the preferred scope of the present invention (hydrogen atom, an alkyl group having 1 to 3 carbons, allyl group, carbobenzoyloxy group, or group represented by the general formula (2) or (3)). It is most preferable that (the other) one be a hydrogen atom because the effect will be the best. In this case, R₁ is not a hydrogen atom (glycine derivative).

Specific examples of the corresponding preferred α-amino acid derivatives may include, for example, *N*-methyl-L-alanine, *N*-methyl-DL-alanine, *N*-methyl-D-alanine, *N*-ethyl-L-alanine, *N*-ethyl-DL-alanine, *N*-ethyl-D-alanine, *N*-*n*-propyl-L-alanine, *N*-*n*-propyl-DL-alanine, *N*-*n*-propyl-D-alanine, *N*-*iso-*propyl-L-alanine, *N-iso-*propyl-DL-alanine, *N*-*iso-*propyl-D-alanine, *N*-methyl-L-serine, *N*-methyl-DL-serine, *N-*methyl-D-serine, *N*-ethyl-L-serine, *N*-ethyl-DL-serine, *N*-ethyl-D-serine, *N*-*n*-propyl-L-serine, *N*-*n*-propyl-DL-serine, *N*-*n*-propyl-D-serine, *N*-*iso-*propyl-L-serine, *N*-*iso*-propyl-DL-serine, and *N*-*iso*-propyl-D-serine.

*N*-methyl-L-alanine, *N*-methyl-DL-alanine, *N*-methyl-D-alanine, *N*-ethyl-L-alanine, *N*-ethyl-DL-alanine, *N*-ethyl-D-alanine, *N*-methyl-L-serine, *N*-methyl-DL-serine, *N*-methyl-D-serine, *N*-ethyl-L-serine, *N*-ethyl-DL-serine, and *N*-ethyl-D-serine are further preferred.

The effect will be good when one of R₂ and R₃ is an allyl group as long as (the other) one is within the preferred scope of the present invention (hydrogen atom, alkyl group having 1 to 3 carbons, allyl group, carbobenzoyloxy group, or group represented by the general formula (2)or(3)). It is most preferable that (the other) one be a hydrogen atom because the effect will be the best. In this case, R₁ is not a hydrogen atom (glycine derivative).

Specific examples of the preferred corresponding α-amino acid derivatives may include, for example, *N*-aryl-L-alanine, *N*-aryl-DL-alanine, *N*-aryl-D-alanine, *N-*aryl-L-serine, *N*-aryl-DL-serine, and *N*-aryl-D-serine.

Preferably, when one of R₂ and R₃ is a carbobenzoyloxy group, (the other) one is a hydrogen atom, alkyl group having 1 to 3 carbons, or aryl group because the effect of the present invention will be good as described above. A hydrogen atom, methyl group, or ethyl group is preferred, and a hydrogen atom is the most preferable, because the effect will be better. As previously mentioned, in this case R₁ is not a hydrogen atom (glycine derivative).

Specific examples of preferred α-amino acid derivatives may include, for example, *N*-carbobenzoyloxy-L-alanine, *N*-carbobenzoyloxy-DL-alanine, *N-*carbobenzoyloxy-D-alanine, *N*-carbobenzoyloxy-L-serine, *N*-carbobenzoyloxy-DL-serine, *N*-carbobenzoyloxy-D-serine, *N*-carbobenzoyloxy-*N*-methyl-L-alanine, *N-*carbobenzoyloxy-*N*-methyl-DL-alanine, *N*-carbobenzoyloxy-*N*-methyl-D-alanine, *N-*carbobenzoyloxy-*N*-methyl-L-serine, *N*-carbobenzoyloxy-*N*-methyl-DL-serine, *N-*carbobenzoyloxy-*N*-methyl-D-serine, *N*-carbobenzoyloxy-*N*-ethyl-L-alanine, *N-*carbobenzoyloxy-*N*-ethyl-DL-alanine, *N*-carbobenzoyloxy-*N*-ethyl-D-alanine, *N-*carbobenzoyloxy-*N*-ethyl-L-serine, *N*-carbobenzoyloxy-*N*-ethyl-DL-serine, and *N-*carbobenzoyloxy-*N*-ethyl-D-serine.

The most preferred examples may include, for example, *N*-carbobenzoyloxy-L-alanine, *N*-carbobenzoyloxy-DL-alanine, *N*-carbobenzoyloxy-D-alanine, *N-*carbobenzoyloxy-L-serine, *N*-carbobenzoyloxy-DL-serine, and *N*-carbobenzoyloxy-D-serine.

X₁, X₂, and X₃ in general formula (2) each independently represents an alkyl group having 1 to 4 carbons, alkoxy group having 1 to 4 carbons, hydroxyl group, amino group, monoalkyl or dialkylamino group having 1 to 4 carbons, fluorine atom, or trifluoromethyl group.

Specific examples of alkyl groups having 1 to 4 carbons may include, for example, methyl, ethyl, *n*-propyl, *iso*-propyl, *n*-butyl, *iso*-butyl, *tert*-butyl, and 1-methylpropyl groups.

Specific examples of alkoxy groups having 1 to 4 carbons may include, for example, methoxy, ethoxy, *n*-propoxy, *iso*-propoxy, *n*-butoxy, *iso*-butoxy, *tert*-butoxy, and 2-methylpropyloxy groups.

Examples of monoalkylamino and dialkylamino groups having 1 to 4 carbons may include, for example, *N*-methylamino, *N*-ethylamino, *N*-*n*-propylamino, N-*iso-*propylamino, *N*-*n-*butylamino, *N*-*iso*-butylamino, *N*-*tert*-butylamino, N-(1-methylpropyl)amino, *N,N*-dimethylamino, N-ethyl-N-methylamino, N-methyl-N-*n-*propylamino, *N*-methyl-*N*-*iso*-propylamino, *N*-*n*-butyl-*N*-methylamino, N-*iso*-butyl-N-methylamino, *N*-*tert*-butyl-*N*-methylamino, *N*-(1-methylpropyl)-*N*-methylamino, N-ethyl-N-methylamino, *N,N*-diethylamino, *N*-ethyl-*N*-*n-*propylamino, N-ethyl-N-*iso-*propylamino, *N*-*n-*butyl-*N*-ethylamino, *N*-*iso*-butyl-*N*-ethylamino, *N-tert-*butyl*-N-*ethylamino, *N*-ethyl-*N*-(1-methylpropyl)amino, *N*-methyl-*N*-*n*-propylamino, *N*-ethyl-*N-n*-propylamino, *N*,*N*-di(*n-*propyl)amino, *N*-*n-*propyl-*N*-*iso*-propylamino, *N-n-butyl-N-n-*propylamino, *N*-*iso*-butyl-*N*-*n-*propylamino, *N*-*tert*-butyl-*N*-*n-*propylamino, *N*-(1-methylpropyl)-*N*-*n-*propylamino,

*N*-methyl-*N*-*iso*-propylamino, *N*-ethyl-*N*-*iso*-propylamino, *N*-*iso*-propyl-*N*-*n-*propylamino, *N*,*N*-di(*iso*-propyl)amino, *N*-*n*-butyl-*N*-*iso*-propylamino, *N*-*iso*-butyl-*N-iso*-propylamino, *N*-*tert*-butyl-*N*-*iso*-propylamino, *N-*(1-methylpropyl)-*N*-*iso-*propylamino, *N*-*n*-butyl-*N*-methylamino, *N*-*n*-butyl-*N*-ethylamino, *N*-*n*-butyl-*N*-*n-*propylamino, *N*-*n*-butyl-*N*-*iso*-propylamino, *N*,*N*-di(*n*-butylamino), *N*-*n*-butyl-*N*-*iso-*butylamino, *N*-*n*-butyl-*N*-*tert*-butylamino, *N*-*n*-butyl-*N*-(1-methylpropyl)amino, *N-iso-*butyl-*N*-methylamino, *N*-*iso*-butyl-*N*-ethylamino, *N*-*iso*-butyl-*N*-*n*-propylamino, *N*-*n-*butyl-*N*-*iso*-propylamino, *N*,*N*-di(*iso*-butylamino), *N*-*iso*-butyl-*N*-*ter* (*1)-butylamino,

*N*-*iso*-butyl-*N*-*tert*-butylamino, *N*-*iso*-butyl-*N*-1-methylpropylamino, *N*-*ter* (*1)-butyl-*N*-methylamino, *N*-ter(*1)-butyl-N-ethylamino, *N*-*ter*(*1)-butyl-*N*-*n-*propylamino, *N*-*ter*(*1)-butyl-*N*-*iso*-propylamino, *N,N*-di(*ter*(*1)-butylamino), *N*-*ter*(*1)-butyl-*N*-*iso-*butylamino, *N*-*n*-butyl-*N*-*tert*-butylamino, *N-ter*(*1)-butyl-*N*-(1-methylpropyl)amino, *N-*methyl-*N*-(1-methylpropyl)amino, *N*-ethyl-*N*-(1-methylpropyl)amino, *N*-(1-methylpropyl)-*N-n-*propylamino, *N*-(1-methylpropyl)-*N*-*iso*-propylamino, *N-n*-butyl-1-methylpropylamino, *N-iso*-butyl-*N*-(1-methylpropyl)amino, *N-tert*-butyl-*N*(1-methylpropyl)amino, and *N*,*N*-di(1-methylpropyl)amino groups.

X₁₋X₃ are preferably alkyl groups having 1 to 4 carbons, alkoxy groups having 1 to 4 carbons, or hydroxyl groups because the effect of the present invention will be good. It is particularly preferred that they be methyl, ethyl, methoxy, ethoxy, or hydroxyl groups because solubility will be better.

In general formula (2), n, m, and p each independently represents an integer of 0 to 3. These integers represent the number of substitutions of X₁ through X₃, and the substitution position can be any position. In terms of the effect of the present invention and solubility, n, m, and p preferably satisfy the expression n + m + p < 4, and most preferably satisfy the expression n = m = p = 0.

In general formula (2), k and q each independently represents integers of 0 to 2. When q is 0, the cyclic alkyl segment of general formula (2) is a cyclopentyl segment corresponding to X₁ through X₃, n, m, and p; when q is 1, the segment is the corresponding cyclohexyl segment; and when q is 2, the segment is the corresponding cycloheptyl segment. In general formula (2), k represents the number of methylene groups up to the bonding position with the cyclic alkyl segment that corresponds to X₁ through X₃, n, m, and p.

General formula (2) preferably has a cyclohexyl segment (q = 1) because the effect of the α-amino acid derivative of the present invention will be good. Specifically, it is preferable to use a cyclohexyl group that corresponds to X₁ through X₃, n, m, and p; a corresponding cyclohexylmethyl group, or a corresponding 2-cyclohexylethyl group. A cyclohexyl group (n = m = p = k =0, q = 1), cyclohexylmethyl group (n = m = p = 0, k = 1, q = 1), or 2-cyclohexylethyl group (n = m = p = 0, k = 2, q = 1) is preferred, and a cyclohexyl group (n = m = p = k = 0, q = 1) is the most preferred because both solubility and the effect will be good.

When one of R₂ and R₃ in general formula (1) is a group represented by general formula (2), (the other) one is preferably a hydrogen atom, alkyl group having 1 to 3 carbons, or aryl group because the effect will be good as previously described. A hydrogen atom, methyl group, or ethyl group is particularly preferred, and a hydrogen atom is the most preferred because the effect will be even better.

Specific examples of corresponding preferred α-amino acid derivatives may include, for example, *N*-cyclohexyl-glycine, *N*-cyclohexyl-L-alanine, *N*-cyclohexyl-DL-alanine, *N*-cyclohexyl-D-alanine, *N*-cyclohexyl-L-serine, *N*-cyclohexyl-DL-serine, *N-*cyclohexyl-D-serine, *N*-cyclohexylmethyl-glycine, *N*-cyclohexylmethyl-L-alanine, *N-*cyclohexylmethyl-DL-alanine, *N*-cyclohexylmethyl-D-alanine, *N*-cyclohexylmethyl-L-serine, *N*-cyclohexylmethyl-DL-serine, *N*-cyclohexylmethyl-D-serine, *N*-2-cyclohexylethyl-glycine, *N*-2-cyclohexylethyl-L-alanine, *N-2*-cyclohexylethyl-DL-alanine, *N*-2-cyclohexylethyl-D-alanine, *N*-2-cyclohexylethyl-L-serine, *N*-2-cyclohexylethyl-DL-serine, *N*-2-cyclohexyl-D-serine, and methylated and ethylated forms thereof.

*N*-cyclohexyl-glycine, *N*-cyclohexyl-L-alanine, *N*-cyclohexyl-DL-alanine, *N-*cyclohexyl-D-alanine, *N*-cyclohexyl-L-serine, *N*-cyclohexyl-DL-serine, and *N-*cyclohexyl-D-serine are further preferred.

In general formula (3), X₁ through X₃, k, n, m, and p are the same similarly to general formula (2). When k is 0, general formula (3) expresses a benzenesulfonyl group that corresponds to X₁ through X₃, n, m, and p; when k is 1, the formula expresses a corresponding benzoylsulfonyl group; and when k is 2, the formula expresses a corresponding phenylethylsulfonyl group.

X₁ through X₃ are preferably hydroxyl, methyl, ethyl, propyl, methoxy, ethoxy, or propoxy groups because the effect and solubility of the present invention will be good. A methoxy group is the most preferable.

n, m, and p are preferably n + m + p < 4 because the effect of the present invention will be good; more preferably n + m + p < 2 because solubility will be good; and most preferably n = m = p = 0 because the effect will be the best. In terms of ease of synthesis of the present invention, k is preferably 0 or 1, and most preferably 0, because the effect will be good.

Specific examples of the corresponding preferred group represented by general formula (3) are benzenesulfonyl, 2-methoxybenzenesulfonyl, 3-methoxybenzenesulfonyl, 4-methoxybenzenesulfonyl, 4-ethoxybenzenesulfonyl, 3-ethoxybenzenesulfonyl, 2-ethoxybenzenesulfonyl, 4-propoxybenzenesulfonyl, 3-propoxybenzenesulfonyl, 2-propoxybenzenesulfonyl, 2,4-dimethoxybenzenesulfonyl, 3,4-dimethoxybenzenesulfonyl, 3,4,5-trimethoxybenzenesulfonyl, 2-hydroxybenzenesulfonyl, 3-hydroxybenzenesulfonyl, 4-hydroxybenzenesulfonyl, 2,4-dihydroxybenzenesulfonyl, 3,4-dihydroxybenzenesulfonyl, 3,4,5-trihydroxybenzenesulfonyl, 2-hydroxy-4-methoxybenzenesulfonyl, 3-hydroxy-4-methoxybenzenesulfonyl, 4-hydroxy-3-methoxybenzenesulfonyl, 2-methylbenzenesulfonyl, 3-benzenesulfonyl, 4-methylbenzenesulfonyl, 2-ethylbenzenesulfonyl, 3-ethylbenzenesulfonyl, 4-ethylbenzenesulfonyl, 2-propylbenzenesulfonyl, 3-propylbenzenesulfonyl, 4-propylbenzenesulfonyl,

benzenesulfonyl, 2-methoxybenzoylsulfonyl, 3-methoxybenzoylsulfonyl, 4-methoxybenzoylsulfonyl, 4-ethoxybenzoylsulfonyl, 3-ethoxybenzoylsulfonyl, 2-ethoxybenzoylsulfonyl, 4-propoxybenzoylsulfonyl, 3-propoxybenzoylsulfonyl, 2-propoxybenzoylsulfonyl, 2,4-dimethoxybenzoylsulfonyl, 3,4-dimethoxybenzoylsulfonyl, 3,4,5-trimethoxybenzoylsulfonyl, 2-hydroxybenzoylsulfonyl, 3-hydroxybenzoylsulfonyl, 4-hydroxybenzoylsulfonyl, 2,4-dihydroxybenzoylsulfonyl, 3,4-dihydroxybenzoylsulfonyl, 3,4,5-trihydroxybenzoylsulfonyl, 2-hydroxy-4-methoxybenzoylsulfonyl, 3-hydroxy-4-methoxybenzoylsulfonyl, 4-hydroxy-3-methoxybenzoylsulfonyl, 2-methylbenzoylsulfonyl, 3-methylbenzoylsulfonyl, 4-methylbenzoylsulfonyl, 2-ethylbenzoylsulfonyl, 3-ethylbenzoylsulfonyl, 4-ethylbenzoylsulfonyl, 2-propylbenzoylsulfonyl, 3-propylbenzoylsulfonyl, 4-propylbenzoylsulfonyl,

phenylethylsulfonyl, 4-methoxyphenylethylsulfonyl, 3-methoxyphenylethylsulfonyl, 2-methoxyphenylethylsulfonyl, 4-ethoxyphenylethylsulfonyl, 3-ethoxyphenylethylsulfonyl, 2-ethoxyphenylethylsulfonyl, 4-propoxyphenylethylsulfonyl, 3-propoxyphenylethylsulfonyl, 2-propoxyphenylethylsulfonyl, 2,4-dimethoxyphenylethylsulfonyl, 3,4-dimethoxyphenylethylsulfonyl, 3,4,5-trimethoxyphenylethylsulfonyl, 2-hydroxyphenylethylsulfonyl, 3-hydroxyphenylethylsulfonyl, 4-hydroxyphenylethylsulfonyl, 2,4-dihydroxyphenylethylsulfonyl, 3,4-dihydroxyphenylethylsulfonyl, 3,4,5-trihydroxphenylethylsulfonyl, 2-hydroxy-4-methoxyphenylethylsulfonyl, 3-hydroxy-4-methoxyphenylethylsulfonyl, 4-hydroxy-3-methoxyphenylethylsulfonyl, 2-methylphenylethylsulfonyl, 3-methylphenylethylsulfonyl, 4-methylphenylethylsulfonyl, 2-ethylphenylethylsulfonyl, 3-ethylphenylethylsulfonyl, 4-ethylphenylethylsulfonyl, 2-propylphenylethylsulfonyl, 3-propylphenylethylsulfonyl, and 4-propylphenylethylsulfonyl groups.

Examples of further preferred groups represented by general formula (3) may include, for example, benzenesulfonyl, 4-methoxybenzenesulfonyl, 3-methoxybenzenesulfonyl, and 2-methoxybenzenesulfonyl groups. A benzenesulfonyl group is the most preferred as the group represented by general formula (3).

When one of R₂ and R₃ in general formula (1) is a group represented by general formula (3), (the other) one is preferably a hydrogen atom, an alkyl group having 1 to 3 carbons, or an aryl group because the effect of the invention will be good. A hydrogen atom, methyl group, or ethyl group is particularly preferred, and a hydrogen atom is the most preferred, because the effect will be better.

Examples of the corresponding preferred α-amino acid derivative may include, for example, *N*-benzenesulfonyl-glycine, *N*-4'-methoxybenzenesulfonyl-glycine, *N*-3'-methoxybenzenesulfonyl-glycine, *N*-2'-methoxybenzenesulfonyl-glycine, *N-*benzoylsulfonyl-glycine, *N*-4'-methoxybenzoylsulfonyl-glycine, *N*-3'-methoxybenzoylsulfonyl-glycine, *N*-2'-methoxybenzoylsulfonyl-glycine, *N-*phenylethylsulfonyl-glycine, *N*-4'-methoxyphenylethylsulfonyl-glycine, *N*-3'-methoxyphenylethylsulfonyl-glycine, *N*-2'-methoxyphenylethylsulfonyl-glycine.

*N*-benzenesulfonyl-D-alanine, *N*-4'-methoxybenzenesulfonyl-D-alanine, *N-3'-*methoxybenzenesulfonyl-D-alanine, *N-2'*-methoxy-benzenesulfonyl-D-alanine, *N-*benzoylsulfonyl-D-alanine, *N*-4'-methoxybenzoylsulfonyl-D-alanine, *N*-3'-methoxybenzoylsulfonyl-D-alanine, *N*-2'-methoxybenzoylsulfonyl- D-alanine, *N-*phenylethylsulfonyl-D-alanine, *N*-4'-methoxyphenylethylsulfonyl- D-alanine, *N*-3'-methoxyphenylethylsulfonyl- D-alanine, *N*-2'-methoxyphenylethylsulfonyl D-alanine,

*N*-benzenesulfonyl-DL-alanine, *N*-4'-methoxybenzenesulfonyl-DL-alanine, *N*-3'-methoxybenzenesulfonyl-DL-alanine, *N*-2'-methoxybenzenesulfonyl-DL-alanine, *N-*benzoylsulfonyl-DL-alanine, *N*-4'-methoxybenzoylsulfonyl-DL-alanine, *N*-3'-methoxybenzoylsulfonyl-DL-alanine, *N*-2'-methoxybenzoylsulfonyl-DL-alanine, *N-*phenylethylsulfonyl-DL-alanine, N-4'-methoxyphenylethylsulfonyl-DL-alanine, N-3'-methoxyphenylethylsulfonyl-DL-alanine, *N*-2'-methoxyphenylethylsulfonyl-DL-alanine,

*N*-benzenesulfonyl-L-alanine, *N*-4'-methoxybenzenesulfonyl-L-alanine, *N-3'-*methoxybenzenesulfonyl-L-alanine, *N-2'*-methoxybenzenesulfonyl-L-alanine, *N-*benzoylsulfonyl-L-alanine, *N*-4'-methoxybenzoylsulfonyl-L-alanine, *N*-3'-methoxybenzoylsulfonyl-L-alanine, *N*-2'-methoxybenzoylsulfonyl-L-alanine, N-phenylethylsulfonyl-L-alanine, N-4'-methoxyphenylethyl sulfonyl-L-alanine, N-3'-methoxyphenylethylsulfonyl-L-alanine, *N*-2'-methoxyphenylethylsulfonyl-L-alanine,

*N*-benzenesulfonyl-D-serine, *N*-4'-methoxybenzenesulfonyl-D-serine, *N*-3'-methoxy-benzenesulfonyl-D-serine, *N*-*2'*-methoxybenzenesulfonyl-D-serine, *N-*benzoylsulfonyl-D-serine, *N*-4'-methoxybenzoylsulfonyl-D-serine, *N*-3'-methoxybenzoylsulfonyl-D-serine, *N*-2'-methoxybenzoylsulfonyl- D-serine, *N-*phenylethylsulfonyl-D-serine, *N*-4'-methoxyphenylethylsulfonyl- D-serine, *N*-3'-methoxyphenylethylsulfonyl-D-serine, *N*-2'-methoxyphenylethylsulfonyl-D-serine,

*N*-benzenesulfonyl-DL-serine, *N*-4'-methoxybenzenesulfonyl-DL-serine, *N-*3'-methoxybenzenesulfonyl-DL-serine, *N-2*'-methoxybenzenesulfonyl-DL-serine, *N-*benzoylsulfonyl-DL-serine, *N*-4'-methoxybenzoylsulfonyl-DL-serine, *N*-3'-methoxybenzoylsulfonyl-DL-serine, *N*-2'-methoxybenzoylsulfonyl-DL-serine, N-phenylethylsulfonyl-DL-serine, N-4'-methoxyphenylethylsulfonyl-DL -serine, N-3'-methoxyphenylethylsulfonyl-DL-serine, *N*-2'-methoxyphenylethylsulfonyl-DL-serine,

*N*-benzenesulfonyl-L-serine, *N*-4'-methoxybenzenesulfonyl-L-serine, N-3'-methoxybenzenesulfonyl-L-serine, *N*-2'-methoxybenzenesulfonyl-L -serine, N-benzoylsulfonyl-L-serine, *N*-4'-methoxybenzoylsulfonyl-L-serine, *N*-3'-methoxybenzoylsulfonyl-L-serine, *N*-2'-methoxybenzoylsulfonyl-L-serine, N-phenylethylsulfonyl-L-serine, N-4'-methoxyphenylethylsulfonyl-L-serine, N-3'-methoxyphenylethylsulfonyl-L-serine, and *N*-2'-methoxyphenylethylsulfonyl-L-serine, and N-methylated and N-ethylated forms thereof.

Particularly preferred α-amino acid derivatives are *N*-benzenesulfonyl-glycine, *N*-4'-methoxybenzenesulfonyl-glycine, *N*-3'-methoxybenzenesulfonyl-glycine, *N*-2'-methoxybenzenesulfonyl-glycine, *N*-benzenesulfonyl-D-alanine, *N*-4'-methoxybenzenesulfonyl-D-alanine, *N*-3'-methoxybenzenesulfonyl-D-alanine, *N-2'-*methoxybenzenesulfonyl-D-alanine, *N*-benzenesulfonyl-DL-alanine, N-4'-methoxybenzenesulfonyl-DL-alanine, *N*-3'-methoxybenzenesulfonyl-DL-alanine, *N-2'-*methoxybenzenesulfonyl-DL-alanine, *N*-benzenesulfonyl-L-alanine, *N*-4'-methoxybenzenesulfonyl-L-alanine, *N*-3'-methoxybenzenesulfonyl-L-alanine, *N-2'-*methoxybenzenesulfonyl-L-alanine, *N*-benzenesulfonyl-D-serine, *N*-4'-methoxybenzenesulfonyl-D-serine, *N*-3'-methoxybenzenesulfonyl-D-serine, *N*-2'-methoxybenzenesulfonyl-D-serine, *N*-benzenesulfonyl-DL-serine, *N*-4'-methoxybenzenesulfonyl-DL-serine, *N-3'*-methoxybenzenesulfonyl-DL-serine, *N-2'-*methoxybenzenesulfonyl-DL-serine, *N*-benzenesulfonyl-L-serine, *N*-4'-methoxybenzenesulfonyl-L-serine, *N*-3'-methoxybenzenesulfonyl-L-serine, and *N*-2'-methoxybenzenesulfonyl-L-serine.

The most preferred α-amino acid derivatives are *N*-benzenesulfonyl-glycine, *N*-benzenesulfonyl-D-alanine, *N*-benzenesulfonyl-DL-alanine, *N*-benzenesulfonyl-L-alanine, *N*-benzenesulfonyl-D-serine, *N*-benzenesulfonyl-DL-serine, and *N-*benzenesulfonyl-L-serine.

The α-amino acid derivative represented by general formula (1) of the present invention can be a salt or a combination of salts. Although the present invention is not limited to these, examples of the salt combinations may include, for example, alkali metal and alkaline earth metal ions such as sodium, potassium, calcium, zinc, and magnesium; ammonium ions; amine ions such as methylamine, pyridine, trimethylamine, and triethanol amine; acids such as hydrochloric acid, sulfuric acid, phosphoric acid, hydrobromic acid, and alkylsulfuric acids such as methyl sulfuric acid and p-toluenesulfonic acid, as well as acetic acid, lactic acid, maleic acid, fumaric acid, oxalic acid, succinic acid, tartaric acid, and citric acid; and amino acids such as betaine, glycine, alanine, serine, and taurine.

A compound selected from the group consisting of *N*-methyl-L-serine, *N-*methyl-DL-serine, *N*-methyl-D-serine, *N*-ethyl-L-serine, *N*-ethyl-DL-serine, *N*-ethyl-D-serine, *N*-methyl-L-alanine, *N*-methyl-DL-alanine, *N*-methyl-D-alanine, *N*-ethyl-L-alanine, *N*-ethyl-DL-alanine, *N*-ethyl-D-alanine, *N*-carbobenzoyloxy-L-serine, *N-*carbobenzoyloxy-DL-serine, *N*-carbobenzoyloxy-D-serine, *N*-carbobenzoyloxy-L-alanine, *N*-carbobenzoyloxy-DL-alanine, *N*-carbobenzoyloxy-D-alanine, *N-*cyclohexyl-glycine, *N*-cyclohexyl-DL-serine, *N*-cyclohexyl-L-serine, *N*-cyclohexyl-D-serine, *N-*cyclohexyl-L-alanine, *N*-cyclohexyl-DL-alanine, *N*-cyclohexyl-D-alanine, *N-*benzenesulfonyl-glycine, *N*-benzenesulfonyl-L-serine, *N*-benzenesulfonyl-DL-serine, *N*-benzenesulfonyl-D-serine, *N*-benzenesulfonyl-L-alanine, *N*-benzenesulfonyl-DL-alanine, and *N*-benzenesulfonyl-L-alanine (*1a), and salts thereof, is the most preferred compound for the α-amino acid derivative of the present invention in terms of solving the object of the present invention in that the effect of inhibiting parakeratosis, pore shrinkage, and preventing/amaliorating rough skin will be the best, solubility in the preparation will be good, and the product will be very safe with no problems such as sensory irritation.

It is a novel feature that the α-amino acid derivatives represented by general formula (1) according to the present invention and salts thereof have a parakeratosis-inhibiting effect, pore-shrinking effect, and rough skin preventing/amaliorating effect.

An α-amino acid derivative represented by general formula (1) having an alkyl group having 1 to 3 carbons, aryl group, or carbobenzoyloxy group can be easily obtained by introducing the corresponding *N*-alkyl, *N*-allyl, or *N*-carbobenzoyloxy group to the corresponding α-amino acid or corresponding analog.

An α-amino acid derivative represented by general formula (1) having a group represented by general formula (2) can be easily obtained by introducing a corresponding group represented by general formula (2) to the corresponding α-amino acid or corresponding analog. It can also be easily obtained by methods such as removing protector groups once the protected corresponding general formula (2) has been introduced, and deriving a compound of general formula (2) after introduction of groups corresponding to general formula (2).

An α-amino acid derivative represented by general formula (1) having a group represented by general formula (3) can be easily obtained by introducing a corresponding group represented by general formula (3) to the corresponding α-amino acid or corresponding analog. The method cited in Japanese Translation of PCT International Application No. 9-504300 is an example. It can also be easily obtained by methods such as removing protector groups after removal of the protected corresponding general formula (3), and deriving a compound of general formula (3) similarly to the introduction of aryl groups to the aromatic ring by Friedel-Crafts reaction once the corresponding general formula (3) has been introduced.

The α-amino acid derivative represented by general formula (1) of the present invention can be easily obtained by reacting the corresponding α-amino acid analog and the corresponding amine. An example is the method disclosed in Japanese Laid Open Patent No. 61-161247. When one of R₂ and R₃ is a hydrogen atom, it is possible to introduce a group corresponding to (the other) group to a corresponding α-amino acid wherein one of the amino groups has been protected. When one of the groups is an alkyl group having 1 to 3 carbons or a group represented by general formula (2), it can be introduced using the corresponding aldehyde. Once R₂ and R₃ or a group corresponding to the same has been introduced to the protected corresponding α-amino acid analog, the amino acid derivative can be obtained by removing the protector groups. It is also possible to introduce R₂ and R₃ or a group corresponding to the same incrementally.

In particular, when the α-amino acid derivative represented by general formula (1) according to the present invention is *N*-methyl-DL-alanine, the compound is a conventional material that can be easily synthesized by conventional methods or can be easily obtained as a commercial product from Sigma-Aldrich Co.

In particular, when the α-amino acid derivative represented by general formula (1) according to the present invention is *N*-methyl-L-alanine or *N*-methyl-L-serine, the compound is a conventional material that can be easily synthesized by conventional methods. For example, it is a known fact that the compound can be obtained by the method of P. Quitt et al. (Helv. Chem. Acta, 327-333, 1963).

In particular, when the α-amino acid derivative represented by general formula (1) according to the present invention is *N*-cyclohexyl-glycine, N-cyclopentyl-glycine, *N*-cycloheptyl-glycine, *N*-cyclohexyl-L-alanine, *N*-cyclopentyl- L-alanine, or *N-*cyclopentyl- L-alanine, the compound is a conventional material that can be easily synthesized by conventional methods. It is known that *N*-cyclohexyl-glycine can be obtained by the method disclosed in Japanese Laid Open Patent No. 57-26656 and above-mentioned Japanese Laid Open Patent No. 61-161247. In addition, it is known that N-cyclopentyl-glycine, *N*-cycloheptyl-glycine, *N*-cyclohexyl-L-alanine, *N-*cyclopentyl- L-alanine, and N-cyclopentyl- L-alanine are compounds obtained by the method disclosed in above-mentioned Japanese Laid Open Patent N. 57-26656.

In particular, when the α-amino acid derivative represented by general formula (1) according to the present invention is *N*-carbobenzoyloxy-DL-serine, N-carbobenzoyloxy-L-serine, *N*-carbobenzoyloxy-D-serine, *N*-carbobenzoyloxy-DL-alanine, *N*-carbobenzoyloxy-L-alanine, or *N*-carbobenzoyloxy-D-alanine, the compound is a conventional material that can be easily synthesized by conventional methods or can be easily obtained as a commercial product from Tokyo Chemical Industry Co., Ltd.

In particular, when the α-amino acid derivative represented by general formula (1) according to the present invention is *N*-benzenesulfonyl-L-serine or *N-*benzenesulfonyl-L-alanine, the compound is a conventional material that can be easily synthesized by conventional methods. For example, the compound can be obtained by the method disclosed in Japanese Translation of PCT International Application No. 9-504300.

The following are typical examples of synthesizing the α-amino acid derivative according to the present invention, but the present invention is not limited to these examples.

### (Synthesis Examples)

### (1) N-benzenesulfonyl-glycine

Ten grams of glycine were dissolved in 120 mL of 1 N sodium hydroxide solution (*2), and 17.4 g of benzenesulfonyl chloride were added dropwise under freezing conditions. 2 N sodium hydroxide solution was added, the pH was adjusted to 9.2, and the mixture was stirred for six hours. After washing with 50 mL of ethyl acetate, hydrochloric acid was added and the pH was adjusted to 2 or less. The mixture was extracted with 500 mL of ethyl acetate, desiccated with magnesium sulfate and filtered, and then concentrated under reduced pressure. The resulting residue was recrystallized using aqueous ethanol to obtain 15.0 g of the desired product.

### (2) N-4'-methoxybenzenesulfonyl-glycine

The desired product was obtained by synthesis using 4-methoxybenzenesulfonyl chloride in place of the benzenesulfonyl chloride in Synthesis Example (1).

### (3) N-3'-methoxybenzenesulfonyl-glycine

The desired product was obtained by synthesis using 3-methoxybenzenesulfonyl chloride in place of the benzenesulfonyl chloride in Synthesis Example (1).

### (4) N-2'-methoxybenzenesulfonyl-glycine

The desired product was obtained by synthesis using 2-methoxybenzenesulfonyl chloride in place of the benzenesulfonyl chloride in Synthesis Example (1).

### (5) N-cyclohexyl-DL-alanine

Ten grams of ethyl 2-bromopropionate and 16 g of cyclohexylamine were added to 50 mL of ethanol and heated and refluxed for two hours. After cooling in air, the mixture was concentrated under reduced pressure, extracted with 200 mL of ethyl acetate, and rinsed with purified water. The product was desiccated over magnesium sulfate and concentrated. The resulting residue was distilled under reduced pressure to obtain 9.8 g of *N*-cyclohexyl-DL-alanine ethyl ester. The resulting *N*-cyclohexyl-DL-alanine ethyl ester was added to a sodium hydroxide solution (1.68 g sodium hydroxide/100 mL purified water), and THF was added until the solution became uniform. After stirring for three hours at room temperature, the product was neutralized by Amberlite IR120B[H⁺] and concentrated under reduced pressure. The resulting residue was recrystallized to obtain 7.2 g of product.

### (6) N-cyclohexyl-N-methyl-glycine

20 g of ethyl 2-bromoacetate and 15 g of *N*-cyclohexyl-*N*-methylamine were added to 40 mL of ethanol and heated and refluxed for two hours. After cooling in air, the mixture was concentrated under reduced pressure, extracted with 200 mL of ethyl acetate, and rinsed with purified water. The product was desiccated over magnesium sulfate and concentrated. The resulting residue was distilled under reduced pressure to obtain 10.5 g of *N*-cyclohexyl-DL-alanine ethyl ester (*3). The resulting *N*-cyclohexyl-DL-alanine methyl ester was added to sodium hydroxide solution (2.4 g sodium hydroxide/100 mL purified water), and THF was added until the solution became uniform. After stirring for three hours at room temperature, the product was neutralized by Amberlite IR120B[H⁺] and concentrated under reduced pressure. The resulting residue was recrystallized to obtain 5.1 g of product.

The α-amino acid derivative represented by general formula (1) according to the present invention has an excellent parakeratosis-inhibiting function, pore-shrinking function, and rough skin preventing/amaliorating function, as will be later shown. Consequently, one, two, or more compounds selected from the group consisting of α-amino acid derivatives represented by general formula (1) according to the present invention, and salts thereof, and salts thereof (*4) are useful as a parakeratosis inhibitor, pore-shrinking agent, and rough skin preventing/ameliorating agent.

The α-amino acid derivative of the present invention or a salt thereof is contained as an active ingredient and can be used as a parakeratosis inhibitor, pore-shrinking agent, or rough skin preventing/ameliorating agent. This parakeratosis inhibitor, pore-shrinking agent, and rough skin preventing/ameliorating agent is preferably used externally on skin, and, for example, improves conspicuous pores on the nose and cheeks and prevents or abates rough skin when used on the face, improves conspicuous pores and prevents or abates rough skin after hair removal treatment of the legs, and has other effects when used on the body.

The parakeratosis inhibitor agent, pore-shrinking agent, and rough skin preventing/ameliorating agent is a novel and useful application for the α-amino acid derivative of the present invention or a salt thereof, based on the discovery of the above-mentioned novel function.

The parakeratosis inhibitor agent, pore-shrinking agent, and rough skin preventing/amaliorating agent of the present invention is very safe; therefore, they have a very wide application range and can be used in a variety of fields. They are most preferable in fields that include cosmetics containing pharmaceutical external products, pharmaceutical products, and food products.

Moreover, the α-amino acid derivatives of the present invention and salts thereof, which are the parakeratosis inhibitor agent, pore-shrinking agent, and rough skin preventing/ameliorating agent according to the present invention, are not sensory irritants and are each added to an external composition for skin to prepare an external composition for skin having a function such as parakeratosis inhibition, pore shrinkage, or rough skin prevention/abatement.

This external composition for skin containing the α-amino acid derivative and salt thereof of the present invention as a parakeratosis inhibitor, pore-shrinking agent, or rough skin preventing/ameliorating agent according to the present invention is a novel material that can be used as a highly safe external composition that has a parakeratosis-inhibiting effect, pore-shrinking effect, or rough skin - preventing/ameliorating effect.

The external composition for skin according to the present invention can be used in pore-shrinking agents; facial cosmetics for improving conspicuous pores on the nose, cheeks, and the like; and skin external preparations for the body for preventing or ameliorating rough skin, and particularly improving conspicuous pores after hair removal treatment of legs, and the like.

When a parakeratosis inhibitor, pore-shrinking agent, rough skin preventing/ameliorating agent, external composition for skin, or other such composition contains the α-amino acid derivative of the present invention or a salt thereof, the α-amino acid derivative of the present invention or a salt thereof is contained in an amount that is effective for realizing the relevant function. This content is preferably 0.001 to 20.0 mass%, particularly 0.01 to 10.0 mass%, and particularly preferably 0.2 to 5.0 mass%, in relation to the total amount of composition. When a mixture of the α-amino acid derivatives of the present invention or salts thereof is used, the maximum total content is preferably 20.0 mass% or less, particularly 10.0 mass% or less, and particularly preferably 5.0 mass% or less.

Components normally used for external agents for skin such as cosmetics and preparation examples are optionally added as needed to the parakeratosis inhibitor agent, pore-shrinking agent, rough skin preventing/ameliorating agent, external composition for skin, or other composition according to the present invention. Examples of these components may include, for example, oils, surfactants, powders, pigments, water, alcohols, thickeners, chelating agents, silicones, oxidation inhibitors (antioxidants), UV absorbers, humectants, perfumes, various pharmaceutical components, preservatives, neutralizers, and pH regulators.

Of these optional components, specific examples of oils may include, for example, liquid oils and fats such as avocado oil, camellia oil, turtle oil, macadamia nut oil, corn oil, mink oil, olive oil, rapeseed oil, egg yolk oil, rubber oil, persic oil, wheat germ oil, sasanqua oil, castor oil, linseed oil, safflower oil, cottonseed oil, moonflower oil, perilla oil, soy oil, peanut oil, tea seed oil, Japanese nutmeg oil, rice germ oil, tung oil, Japanese foxglove oil, jojoba oil, germ oil, triglycerin, glycerin trioctanoate, and glycerin triisopalmitate; solid oils and fats such as cocoa oil, coconut oil, equine tallow, hydrogenated coconut oil, palm oil, bovine tallow, ovine tallow, hydrogenated bovine tallow, palm seed oil, porcine tallow, vegetable wax seed oil, hydrogenated oil, vegetable wax, and hydrogenated castor oil; waxes such as beeswax, candelilla wax, carnauba wax, lanolin, lanolin acetate, liquid lanolin, cane wax, isopropyl lanolin fatty acid, hexyl laurate, reduced lanolin, jojoba wax, hydrogenated lanolin, polyoxyethylene (POE hereafter) lanolin alcohol ether, POE lanolin alcohol acetate, lanolin fatty acid polyethylene glycol, and POE hydrogenated lanolin alcohol ether; hydrocarbons such as liquid paraffin, ozokerite, squalene, paraffin, sericin, squalene, Vaseline, and microcrystalline wax;

ester oils such as isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, cholesteryl 12-hydroxystearate, ethylene glycol di-2-ethylhexylate, dipentaerythritol fatty acid ester, *N*-alkylglycol monoisostearate, neopentyl glycol dicaprate, diisostearyl malate, glycerin di-2-heptyl undecanoate, trimethylolpropane tri-2-ethylhexylate, trimethylolpropane triisostearate, pentaerythritol tetra-2-ethylhexylate, glycerin tri-2-ethylhexylate, trimethylolpropane triisostearate, cetyl-2-ethylhexanoate, 2-ethylhexyl palmitate, glycerin trimyristate, glyceride tri-2-heptylundecanoate, castor oil fatty acid methyl ester, oleic acid oil, acetoglyceride, 2-heptylundecyl palmitate,

diisopropyl adipate, *N*-lauroyl-L-glutamic acid-2-octyldodecyl ester, di-2-heptylundecyl adipate, di-2-ethylhexyl sebacate, 2-hexyldecyl myristate, 2-hexyldecyl palmitate, 2-hexyldecyl adipate, diisopropyl sebacate, and 2-ethylhexyl succinate; higher fatty acids such as lauric acid, myristic acid, palmitic acid, stearic acid, behenic acid, oleic acid, 12-hydroxystearic acid, undecylenic acid, lanolin fatty acid, isostearic acid, linolic acid, linoleic acid, and eicosapentaenoic acid; straight and branched higher alcohols such as lauryl alcohol, cetyl alcohol, stearyl alcohol, behenyl alcohol, myristyl alcohol, oleyl alcohol, cetostearyl alcohol, monostearyl glycerin ether (batyl alcohol), 2-decyl tetradecinol, lauric alcohol, cholesterol, phytosterol, hexyl dodecanol, isostearyl alcohol, and octyl dodecanol; silicone oils such as dimethyl polysiloxane and methylphenyl polysiloxane; and perfluorocarbons or perfluoropolyethers such as perfluorohexane and triperfluoro-n-butylamine.

Examples of surfactants as anionic surfactants include fatty acid soaps such as raw soap, sodium laurate, and palmitic acid; higher alkyl sulfuric acid ester salts such as sodium laurylsulfate and potassium laurylsulfate; alkyl ether sulfuric acid ester salts such as POE triethanolamine lauryl sulfate and POE sodium lauryl sulfate; N-acylsarcosinic acids such as sodium lauroylsarcosinate; higher fatty acid amide sulfonates such as N-myristoyl-N-methyltaurine sodium and coconut oil fatty acid methyl laurate sodium; phosphoric acid ester salts such as POE stearyl ether phosphate; sulfosuccinates such as sodium monolauroyl monomethanol amide POE sulfosuccinate and sodium lauryl polypropylene glycol sulfosuccinate; alkylbenzenesulfonates such as sodium linear dodecylbenzenesulfonate and triethanolamine linear dodecyl benzenesulfonate;

*N*-acylglutamates such as disodium N-stearoylglutamate and monosodium N-stearoylglutamate; higher fatty acid ester sulfuric acid ester salts such as hydrogenated coconut oil fatty acid glycerin sodium sulfate; and sulfated oils such as Turkey red oil; and anionic surfactants such as POE alkyl ether carboxylic acid, POE alkyl allyl ether carboxylic acid, higher fatty acid ester sulfonate, secondary alcohol sulfuric acid ester salt, higher fatty acid alkylol amide sulfuric acid ester salt, lauroylmonoethanolamide sodium succinate, and casein sodium. Examples of cationic surfactants include alkyl trimethylammonium salts such as stearyltrimethylammonium chloride and lauryltrimethylammonium chloride; dialkylmethylammonium salts such as distearyldimethylammonium chloride salt; and alkylpyridinium salts such as cetylpyridinium chloride; as well as alkyltetraammonium salts, alkyldimethylbenzoylammonium salts, alkylisoquinolinium salts, dialkylmorpholinium salts, POE alkylamines, alkylamine salts, polyamine aliphatic acid derivatives, acyl alcohol fatty acid derivatives, and benzalkonium chloride.

Examples of amphoteric surfactants include imidazoline amphoteric surfactants such as 2-cocoyl-2-imidazolinium hydroxide-1-carboxyethyloxy disodium salt and betaine surfactants such as amidobetaine and sulfobetaine.

Examples of hydrophobic nonionic surfactants include sorbitan fatty acid esters such as sorbitan monooleate, sorbitan monoisostearate, sorbitan monolaurate, sorbitan monopalmitate, and sorbitan trioleate; glycerin polyglycerin fatty acids such as mono-cottonseed oil fatty acid glycerin, glycerin monostearate, glycerin sesquioleate, and glycerin malate monostearate; and propylene glycol fatty acid esters such as propylene glycol monostearate; hydrogenated castor oil derivatives, glycerin alkyl ether, and POE-methylpolysiloxane copolymers.

Examples of hydrophilic nonionic surfactants include POE sorbitan aliphatic acid esters such as POE sorbitan monooleate and POE sorbitan monostearate; POE sorbitol fatty acid esters such as POE sorbitol monolaurate, POE sorbitol monooleate, and POE sorbitol monostearate; POE glycerin fatty acid esters such as POE glycerin monooleate and POE glycerin distearate; POE fatty acid esters such as POE monooleate, POE distearate, and POE monodioleate; POE alkyl ethers such as POL lauryl ether, POE oleyl ether, and POE cholestanol ester; POE alkylphenyl ethers such as POE octylphenyl ether and POE nonylphenyl ether;

POE-polyoxypropylene (POP hereafter) alkyl ethers such as POE-POP monobutyl ether, POE-POP cetyl ether, and POE-POP glycerin ether; POE castor oil hydrogenated castor oil derivatives such as POE castor oil, POE hydrogenated castor oil, POE hydrogenated castor oil monoisostearate, and POE hydrogenated castor oil maleate; POE beeswax lanolin derivatives such as POE sorbitol beeswax; alkanolamides such as coconut oil fatty acid diethanolamide and fatty acid isoproponolamide; as well as hydrophilic nonionic surfactants such as POE propylene glycol fatty acid ester, POE fatty acid amide, POE alkylamine, sucrose fatty acid ester, and alkyl ethoxydimethylaminoxide.

Examples of powders are mica, talc, kaolin, sericite (fine grained mica), muscovite, phlogopite, synthetic mica, lepidolite, biotite, lithia mica, synthetic mica (*5), calcium carbonate, magnesium carbonate, silicic anhydride (silica), aluminum silicate, barium silicate, calcium silicate, magnesium silicate, strontium silicate, aluminum oxide, barium sulfate, Indian red, yellow iron oxide, black iron oxide, cobalt oxide, ultramarine, Prussian blue, titanium oxide, zinc oxide, mica titanium (titanium oxide-coated mica), aluminum foil, bismuth oxychloride, boron nitride, red No. 228, red No. 226, blue No. 404, polyethylene powder, polymethyl methacrylate powder, polyamide resin powder (nylon powder), cellulose powder, organopolysiloxane elastomer, aluminum powder, and copper powder.

Examples of alcohols may include, for example, lower alcohols such as methanol, ethanol, propanol, isopropanol, and the like; cholesterol; sitosterol; and lanosterol.

Examples of thickeners are vegetable polymers such as gum arabic, tragacanth gum, galactan, carob gum, guar gum, carrageenan, pectin, agar, and starch (corn, wheat, potato, rice); microbiological polymers such as dextran and pullulan; starch polymers such as carboxymethyl starch and methyl hydroxypropyl starch; animal polymers such as collagen, casein, and gelatin; cellulose polymers such as methyl cellulose, nitrocellulose, ethyl cellulose, hydroxyethyl cellulose, sodium sulfate cellulose, hydroxypropyl cellulose, carboxymethyl cellulose, and crystalline cellulose; alginic acid polymers such as sodium alginate and propylene glycol alginate; vinyl polymers such as polyvinyl methyl ether and carboxyvinyl polymer; acrylic polymers such as POE polymers, POE polyoxypropylene copolymer polymers, sodium polyacrylate, polyacrylamide; polyethylene imine; cationic polymers; bentonite; aluminum magnesium silicate; laponite; hectorite; and water-soluble polymers such as the inorganic water-soluble polymer of anhydrous silicic acid.

Examples of chelating agents may include, for example, citramalic acid, agaric acid, glyceric acid, shikimic acid, hinokitiol, gallic acid, tannic acid, caffeic acid, ethylenediaminetetraacetic acid, ethylene glycol diamine tetraacetic acid, diethylene triamine pentaacetic acid, phytic acid, polyphosphoric acid, and metaphosphoric acid, as well as analogs and alkali metal salts and carboxylic acid esters thereof.

Examples of UV absorbers may include, for example, benzoic acid UV absorbers such as paraaminobenzoic acid; anthranilic acid UV absorbers such as methyl anthranyl; salicylic acid UV absorbers such as octyl salicylate; cinnamic acid UV absorbers such as isopropyl paramethoxycinnamic acid and octyl paramethoxycinnamic acid; and UV absorbers such as urocanic acid and ethyl urocanate.

Examples of humectants may include, for example, polyethylene glycol (PEG hereafter), propylene glycol, dipropylene glycol, 1,3-butylene glycol, glycerin, diglycerin, xylitol, maltitol, maltose, D-mannitol, glucose, fructose, sodium chondroitin sulfate, sodium hyaluronic acid, sodium lactate, glucosamine, and cyclodextrin.

Examples of pharmaceutical components that can be added are vitamins such as vitamin A oil, retinol, retinol palmitate, pyridoxine hydrochloride, benzoyl nicotinate, nicotinamide, dl-α-tocopherol nicotinate, magnesium ascorbic phosphate, vitamin D2, dl-α-tocopherol, pantothenic acid, and biotin; anti-inflammatory agents such as azulene and glycyrrhizic acid; skin whiteners such as arbutin; hormones such as estradiol; astringents such as zinc oxide and tannic acid; refrigerants such as L-menthol and camphor; lysozyme hydrochloride; pyridoxine hydrochloride; and sulfur. It is also possible to add a variety of extracts having diverse drug effects. Examples may include, for example, dokudami extract, phellodendron extract, glycyrrhiza extract, peony root extract, moutan bark extract, luffa extract, saxifrage extract, eucalyptus extract, clove flower extract, horse chestnut extract, cornflower extract, algae extract, and thyme extract.

Examples of preservatives may include, for example, benzoic acid, salicylic acid, paraoxybenzoic acid esters (methyl paraben, ethyl paraben, butyl paraben), sorbic acid, parachlorometacresol, hexachlorophene, benzalkonium chloride, chlorhexidine chloride, trichlorocarbanilide, photosensitizers, and phenoxyethanol.

Examples of other additives that can be used in preparation examples of the present invention are neutralizers such as 2-amino-2-methyl-1-propanol, 2-amino-2-methyl-1,3-propanediol, potassium hydroxide, potassium hydroxide (*6), triethanolamine, and sodium carbonate; pH regulators such as lactic acid, citric acid, glycolic acid, succinic acid, tartaric acid, malic acid, sodium bicarbonate, and ammonium bicarbonate; and antioxidants such as ascorbic acid, α-tocopherol, and carotenoids.

The above-mentioned components are examples, and the present invention is not limited to these examples. These components can be added in any combination in accordance with a composition that conforms to a predetermined form.

The composition of the present invention, such as a parakeratosis inhibitor, pore-shrinking agent, rough skin preventing/ameliorating agent, and external composition for skin, can be widely used in the form of a pharmaceutical product, a pharmaceutical external product (ointment, dentifrice), or a cosmetic (fundamental cosmetic such as a face wash, milky lotion, cream, gel, essence (clarifier), pack, or mask; basic skin care products; makeup such as foundation or lipstick; oral cosmetic; perfume; hair product; body cosmetic; and the like). The composition of the present invention, such as a parakeratosis inhibitor, pore-shrinking agent, rough skin preventing/ameliorating agent, or external composition for skin, is not limited to these forms.

The dosage form can be a variety of dosage forms, including aqueous solutions, microemulsions, emulsions, oils, gels, ointments, aerosols, water-oil two-layer forms, water-oil-powder three-layer forms, and the like.

By using the composition of the present invention, such as the parakeratosis inhibitor agent, pore-shrinking agent, rough skin preventing/ameliorating agent, or external composition for skin, it is possible to prevent parakeratosis and maintain or improve skin to a healthy state, and to further shrink pores to provide skin with a youthful and fresh appearance without conspicuous pores.

### EXAMPLES

The present invention will now be described using the following examples. Unless otherwise stated, the amounts added are by mass%.

### [Example 1] Parakeratosis-inhibiting effect tests

Aqueous solutions (containing 30 mass% of ethanol) of primarily 3 mass% of an α-amino acid derivative or salt thereof were prepared as the evaluation sample. The pH was regulated by hydrochloric acid or sodium hydroxide so as to be 7.0 to 7.5. When sample solubility was low, the solution was prepared accordingly.

100 µL of 10 mass% oleic acid solution (solvent: ethanol) were applied to the back of a hairless mouse (HR-1; Hoshino Laboratory Animals). Then the sample solution (α-amino acid derivative, and the like) was applied 100 µL at a time. This procedure was continued for three days. The following day the status of the back skin was observed with a CCD camera, and the rough skin status (peeling and redness of the stratum corneum) was evaluated. The status of the skin was evaluated in 0.25-point intervals, with skin where the control (control aqueous solution) had been applied being 2.0 and skin where there was no redness being 0.0. At the same time, the stratum corneum at the back of the hairless mouse was peeled off with tape, the nuclei were stained by hematoxylin, and the degree of parakeratosis was observed and evaluated by a parakeratosis value within a range of 1.0 to 3.0 (in 0.25 increments). An increase in the value indicated that there were many nucleated cells in the stratum corneum, i.e., that parakeratosis was advanced. The results are shown in Table 1.

**[Table 1]**

| Sample | Concentration (mass%) | Macroscopic evaluation (average of four animals) | Parakeratosis value (average of four animals) |
|---|---|---|---|
| Aqueous control solution | | 2.0 | 2.0 |
| *N*-methyl-D-serine | 3 | 1.4 | 1.5 |
| *N*-methyl-L-serine | 3 | 1.3 | 1.2 |
| *N*-methyl-DL-serine | 3 | 1.2 | 1.2 |
| *N*-methyl-D-alanine | 3 | 1.2 | 1.3 |
| *N*-methyl-L-alanine | 3 | 1.4 | 1.4 |
| *N*-methyl-DL-alanine | 3 | 1.4 | 1.4 |
| *N*-ethyl-D-serine | 3 | 1.3 | 1.3 |
| *N*-ethyl-L-serine | 3 | 1.4 | 1.4 |
| *N*-ethyl-DL-serine | 3 | 1.5 | 1.5 |
| *N*-ethyl-D-alanine | 3 | 1.6 | 1.7 |
| *N*-ethyl-L-alanine | 3 | 1.5 | 1.4 |
| *N*-ethyl-DL-alanine | 3 | 1.6 | 1.5 |
| *N*-carbobenzoyloxy-D-serine | 3 | 1.2 | 1.2 |
| *N*-carbobenzoyloxy-L-serine | 3 | 1.2 | 1.1 |
| *N*-carbobenzoyloxy-DL-serine | 3 | 1.2 | 1.2 |
| *N*-carbobenzoyloxy-DL-alanine | 3 | 1.3 | 1.2 |
| *N*-carbobenzoyloxy-L-alanine | 3 | 1.2 | 1.3 |
| *N*-carbobenzoyloxy-D-alanine | 3 | 1.2 | 1.1 |
| N-cyclohexyl-glycine | 3 | 1.2 | 1.1 |
| *N*-cyclohexyl-DL-serine | 3 | 1.2 | 1.2 |
| *N*-cyclohexyl-D-serine | 3 | 1.3 | 1.2 |
| *N*-cyclohexyl-L-serine | 3 | 1.3 | 1.2 |
| *N*-cyclohexyl-DL-alanine | 3 | 1.3 | 1.4 |
| *N*-cyclohexyl-D-alanine | 3 | 1.3 | 1.4 |
| *N*-cyclohexyl-L-alanine | 3 | 1.4 | 1.3 |
| N-benzenesulfonyl-glycine | 3 | 1.2 | 1.2 |
| *N*-benzenesulfonyl-L-serine | 3 | 1.2 | 1.2 |
| *N*-benzenesulfonyl-D-serine | 3 | 1.3 | 1.2 |
| *N*-benzenesulfonyl-DL-serine | 3 | 1.2 | 1.2 |
| *N*-benzenesulfonyl-D-alanine | 1.5 | 1.3 | 1.4 |
| *N*-benzenesulfonyl-L-alanine | 1.5 | 1.2 | 1.3 |
| *N*-benzenesulfonyl-DL-alanine | 1.5 | 1.4 | 1.5 |
| *N*-acetyl-glycine (Comparative Example) | 3 | 1.9 | 2.0 |
| Phosphonomethyl-glycine (Comparative Example) | 1 | 1.9 | 2.1 |
| Hippuric acid (Comparative Example) | 1 | 1.9 | 2.0 |

As is clear from Table 1, a rough skin preventing effect, based on stratum corneum peeling and redness, was seen with *N*-methyl-L-serine, *N*-methyl-DL-serine, *N*-methyl-D-serine, *N*-ethyl-L-serine, *N*-ethyl-DL-serine, *N*-ethyl-D-serine, *N*-methyl-L-alanine, *N*-methyl-DL-alanine, *N*-methyl-D-alanine, *N*-ethyl-L-alanine, *N*-ethyl-DL-alanine, *N*-ethyl-D-alanine, *N*-carbobenzoyloxy-L-serine, *N*-carbobenzoyloxy-DL-serine, *N*-carbobenzoyloxy-D-serine, *N*-carbobenzoyloxy-L-alanine, N-carbobenzoyloxy-DL-alanine, *N*-carbobenzoyloxy-D-alanine, *N*-cyclohexyl-glycine, *N-*cyclohexyl-DL-serine, *N*-cyclohexyl-L-serine, *N*-cyclohexyl-D-serine, *N*-cyclohexyl-L-alanine, *N*-cyclohexyl-DL-alanine, *N*-cyclohexyl-D-alanine, *N*-benzenesulfonyl-glycine, *N*-benzenesulfonyl-L-serine, *N*-benzenesulfonyl-DL-serine, *N*-benzenesulfonyl-D-serine, *N*-benzenesulfonyl-L-alanine, *N*-benzenesulfonyl-DL-alanine, and *N-*benzenesulfonyl-L-alanine. They also reduced the parakeratosis value. Therefore, the above-mentioned compounds had a parakeratosis-inhibiting effect. On the other hand, an effect was not seen with N-acetyl-glycine, hippuric acid, or phosphonomethyl-glycine, none of which is a derivative of the same α-amino acids within the scope of the present invention.

### [Example 2] Human pore-shrinking effect

An experiment was conducted whereby a sample was applied twice a day for one month to the cheeks of healthy human males in groups of 5 men each. Aqueous solutions (containing 15 mass% of ethanol) of 3 mass% of the α-amino acid derivative or a salt thereof were prepared as the evaluation sample. The pH was regulated by hydrochloric acid or sodium hydroxide so as to be 7.0 to 7.5. When sample solubility was low, the solution was prepared accordingly. The control was an aqueous 15% ethanol solution. The sample aqueous solution and control aqueous solution were applied to one half of the face at a time.

A replica was collected before and after continuous application, and changes in the shape of the pores at the same site were observed using a three-dimensional laser scanning microscope. The size of the pores was macroscopically evaluated in 13 steps of 1 through 13 (with the pores becoming larger as the numbers became larger), the difference in the ratings before and after application (after application - before application) was calculated, and the efficacy of each sample was studied using this difference as the replica assessment. The results are shown in Table 2.

**[Table 2]**

| Sample | Concentration (mass%) | Replica assessment (average of n = 5) |
|---|---|---|
| Aqueous control solution | | 0.3 |
| *N*-methyl-D-serine | 3 | -1.5 |
| *N*-methyl-L-serine | 3 | -1.2 |
| *N*-methyl-DL-serine | 3 | -0.7 |
| *N*-methyl-D-alanine | 3 | -0.7 |
| *N*-methyl-L-alanine | 3 | -0.8 |
| *N*-methyl-DL-alanine | 3 | -0.9 |
| *N*-ethyl-D-serine | 3 | -1.2 |
| *N*-ethyl-L-serine | 3 | -1.2 |
| *N*-ethyl-DL-serine | 3 | -1.3 |
| *N*-ethyl-D-alanine | 3 | -0.5 |
| *N*-ethyl-L-alanine | 3 | -0.4 |
| *N*-ethyl-DL-alanine | 3 | -0.5 |
| *N*-carbobenzoyloxy-D-serine | 3 | -1.3 |
| *N*-carbobenzoyloxy-L-serine | 3 | -1.2 |
| *N*-carbobenzoyloxy-DL-serine | 3 | -1.3 |
| *N*-carbobenzoyloxy-DL-alanine | 3 | -1.0 |
| *N*-carbobenzoyloxy-L-alanine | 3 | -0.9 |
| *N*-carbobenzoyloxy-D-alanine | 3 | -0.8 |
| N-cyclohexyl-glycine | 3 | -1.5 |
| *N*-cyclohexyl-DL-serine | 3 | -1.4 |
| *N*-cyclohexyl-D-serine | 3 | -1.3 |
| *N*-cyclohexyl-L-serine | 3 | -1.4 |
| *N*-cyclohexyl-DL-alanine | 3 | -1.4 |
| *N*-cyclohexyl-D-alanine | 3 | -1.3 |
| *N*-cyclohexyl-L-alanine | 3 | -1.2 |
| *N*-benzenesulfonyl-glycine | 3 | -1.3 |
| *N*-benzenesulfonyl-L-serine | 3 | -1.2 |
| *N*-benzenesulfonyl-D-serine | 3 | -1.1 |
| *N*-benzenesulfonyl-DL-serine | 3 | -1.3 |
| *N*-benzenesulfonyl-D-alanine | 1.5 | -1.2 |
| *N*-benzenesulfonyl-L-alanine | 1.5 | -0.9 |
| *N*-benzenesulfonyl-DL-alanine | 1.5 | -0.8 |

As is clear from Table 2, a pore-shrinking effect was seen with *N*-methyl-L-serine, *N*-methyl-DL-serine, *N*-methyl-D-serine, *N*-ethyl-L-serine, *N*-ethyl-DL-serine, *N-*ethyl-D-serine, *N*-methyl-L-alanine, *N*-methyl-DL-alanine, *N*-methyl-D-alanine, *N*-ethyl-L-alanine, *N*-ethyl-DL-alanine, *N*-ethyl-D-alanine, *N*-carbobenzoyloxy-L-serine, *N-*carbobenzoyloxy-DL -serine, *N*-carbobenzoyloxy-D-serine, *N*-carbobenzoyloxy-L-alanine, *N*-carbobenzoyloxy-DL-alanine, *N*-carbobenzoyloxy-D-alanine, *N*-cyclohexyl-glycine, *N*-cyclohexyl-DL-serine, *N*-cyclohexyl-L-serine, *N*-cyclohexyl-D-serine, *N-*cyclohexyl-L-alanine, *N*-cyclohexyl-DL-alanine, *N*-cyclohexyl-D-alanine, *N-*benzenesulfonyl-glycine, *N*-benzenesulfonyl-L-serine, *N*-benzenesulfonyl-DL-serine, *N*-benzenesulfonyl-D-serine, *N*-benzenesulfonyl-L-alanine, *N*-benzenesulfonyl-DL-alanine, and *N*-benzenesulfonyl-L-alanine.

### [Example 3] Inhibiting effect against rough skin induced by oleic acid application

In order to investigate the inhibiting effect of α-amino acid derivatives and salts thereof against rough skin induced by oleic acid application, the transepidermal water loss (TEWL value) before and after application was measured, the difference was compared with the control (control aqueous solution), and the effect was determined. Sample preparation and the application method were in accordance with Example 1. The TEWL was determined using the TM21 0 TEWA Meter (Courage + Khazaka Electronic GmBH).

100 µL of 10 mass% oleic acid (solvent: ethanol) was applied to the back of hairless mice (HR-1, four mice per group). Then a sample solution (α-amino acid derivatives, and the like) was applied 100 µL at a time. This procedure was continued for three days. The following day, the TEWL value of the back was determined and the values of the four animals were averaged. The results are shown in Table 3. Rough skin became worse as the ΔTEWL value increased.

**[Table 3]**

| Sample | Concentration (mass%) | ΔTEWL value |
|---|---|---|
| Aqueous control solution | | 12.0 |
| *N*-methyl-D-serine | 3 | 8.0 |
| *N*-methyl-L-serine | 3 | 7.9 |
| *N*-methyl-DL-serine | 3 | 8.0 |
| *N*-methyl-D-alanine | 3 | 8.2 |
| *N*-methyl-L-alanine | 3 | 8.3 |
| *N*-methyl-DL-alanine | 3 | 8.1 |
| *N*-ethyl-D-serine | 3 | 7.9 |
| *N*-ethyl-L-serine | 3 | 7.7 |
| *N*-ethyl-DL-serine | 3 | 7.8 |
| *N*-ethyl-D-alanine | 3 | 8.3 |
| *N*-ethyl-L-alanine | 3 | 8.4 |
| *N*-ethyl-DL-alanine | 3 | 8.2 |
| *N*-carbobenzoyloxy-D-serine | 3 | 6.8 |
| *N*-carbobenzoyloxy-L-serine | 3 | 6.7 |
| *N*-carbobenzoyloxy-DL-serine | 3 | 6.7 |
| *N*-carbobenzoyloxy-DL-alanine | 3 | 6.6 |
| *N*-carbobenzoyloxy-L-alanine | 3 | 6.9 |
| *N*-carbobenzoyloxy-D-alanine | 3 | 7.1 |
| N-cyclohexyl-glycine | 3 | 6.4 |
| *N*-cyclohexyl-DL-serine | 3 | 6.7 |
| *N*-cyclohexyl-D-serine | 3 | 6.8 |
| *N*-cyclohexyl-L-serine | 3 | 7.0 |
| *N*-cyclohexyl-DL-alanine | 3 | 6.5 |
| *N*-cyclohexyl-D-alanine | 3 | 6.9 |
| *N*-cyclohexyl-L-alanine | 3 | 7.1 |
| *N*-benzenesulfonyl-glycine | 3 | 6.9 |
| *N*-benzenesulfonyl-L-serine | 3 | 6.8 |
| *N*-benzenesulfonyl-D-serine | 3 | 6.7 |
| *N*-benzenesulfonyl-DL-serine | 3 | 6.9 |
| *N*-benzenesulfonyl-D-alanine | 1.5 | 7.0 |
| *N*-benzenesulfonyl-L-alanine | 1.5 | 7.2 |
| *N*-benzenesulfonyl-DL-alanine | 1.5 | 7.0 |
| N-acetyl-glycine (Comparative Example) | 3 | 14.7 |
| Phosphonomethyl-glycine (Comparative Example) | 1 | 11.4 |
| Hippuric acid (Comparative Example) | 1 | 10.5 |

As is clear from Table 3, the ΔTEWL value was significantly lower than that with the control aqueous solution, and a rough skin preventing/amaliorating effect was seen by application of *N*-methyl-L-serine, *N*-methyl-DL-serine, *N*-methyl-D-serine, *N*-ethyl-L-serine, *N*-ethyl-DL-serine, *N*-ethyl-D-serine, *N*-methyl-L-alanine, *N*-methyl-DL-alanine, *N*-methyl-D-alanine, *N*-ethyl-L-alanine, *N*-ethyl-DL-alanine, *N*-ethyl-D-alanine, *N*-carbobenzoyloxy-L-serine, *N*-carbobenzoyloxy-DL-serine, *N-*carbobenzoyloxy-D-serine, *N*-carbobenzoyloxy-L-alanine, *N*-carbobenzoyloxy-DL-alanine, *N*-carbobenzoyloxy-D-alanine, *N*-cyclohexyl-glycine, *N*-cyclohexyl-DL-serine, *N*-cyclohexyl-L-serine, *N*-cyclohexyl-D-serine, *N*-cyclohexyl-L-alanine, *N*-cyclohexyl-DL-alanine, *N*-cyclohexyl-D-alanine, N-benzenesulfonyl-glycine, *N*-benzenesulfonyl-L-serine, *N*-benzenesulfonyl-DL-serine, *N*-benzenesulfonyl-D-serine, *N-*benzenesulfonyl-L-alanine, *N*-benzenesulfonyl-DL-alanine, and *N*-benzenesulfonyl-L-alanine (*7). On the other hand, an effect was not obtained from N-acetyl-glycine, hippuric acid, and phosphonomethyl-glycine, which are not derivatives of the same α-amino acids within the scope of the present invention.

### [Example 4] Sensory irritation tests

1 mL of a control aqueous solution and the sample aqueous solution prepared in Example 1 were applied using a cotton swab to the left or right cheek, respectively, of a panel of 20 women, the irritating sensation was evaluated every 30 seconds for ten minutes beginning immediately after application, and a final evaluation was reported. The evaluation of the irritating sensation was based on the following four steps of rating criteria, the average rating was calculated, and the average was classified by the following criteria.

### (Rating criteria)

3: Discontinued because of extremely strong irritation, such as stinging sensation, burning sensation, tickling sensation, itching, and the like.
2: Intolerable strong irritation, such as stinging sensation, burning sensation, tickling sensation, itching, and the like.
1: Tolerable with only slight stinging sensation, burning sensation, tickling sensation, itching, and the like.
0: Not particularly irritating.

### (Evaluation criteria)

A: average rating of less than 0.2
B: average rating of 0.2 to less than 1.0
C: average rating of 1.0 to less than 2.0
D: average rating of 2.0 or greater

The results are shown in Table 4.

**[Table 4]**

| Sample | Concentration (mass%) | Evaluation |
|---|---|---|
| Aqueous control solution | | A |
| *N*-methyl-D-serine | 3 | A |
| *N*-methyl-L-serine | 3 | A |
| *N*-methyl-DL-serine | 3 | A |
| *N*-methyl-D-alanine | 3 | A |
| *N*-methyl-L-alanine | 3 | A |
| *N*-methyl-DL-alanine | 3 | A |
| *N*-ethyl-D-serine | 3 | A |
| *N*-ethyl-L-serine | 3 | A |
| *N*-ethyl-DL-serine | 3 | A |
| *N*-ethyl-D-alanine | 3 | A |
| *N*-ethyl-L-alanine | 3 | A |
| *N*-ethyl-DL-alanine | 3 | A |
| *N*-carbobenzoyloxy-D-serine | 3 | A |
| *N*-carbobenzoyloxy-L-serine | 3 | A |
| *N*-carbobenzoyloxy-DL-serine | 3 | A |
| *N*-carbobenzoyloxy-DL-alanine | 3 | A |
| *N*-carbobenzoyloxy-L-alanine | 3 | A |
| *N*-carbobenzoyloxy-D-alanine | 3 | A |
| *N*-cyclohexyl-glycine | 3 | A |
| *N*-cyclohexyl-DL-serine | 3 | A |
| *N*-cyclohexyl-D-serine | 3 | A |
| *N*-cyclohexyl-L-serine | 3 | A |
| *N*-cyclohexyl-DL-alanine | 3 | A |
| *N*-cyclohexyl-D-alanine | 3 | A |
| *N*-cyclohexyl-L-alanine | 3 | A |
| *N*-benzenesulfonyl-glycine | 3 | A |
| *N*-benzenesulfonyl-L-serine | 3 | A |
| *N*-benzenesulfonyl-D-serine | 3 | A |
| *N*-benzenesulfonyl-DL-serine | 3 | A |
| *N*-benzenesulfonyl-D-alanine | 1.5 | A |
| *N*-benzenesulfonyl-L-alanine | 1.5 | A |
| *N*-benzenesulfonyl-DL-alanine | 1.5 | A |
| β-alanine (Comparative Example) | 3.0 | D |

There were no problems with sensory irritation, and application was very safe with *N*-methyl-L-serine, *N*-methyl-DL-serine, *N*-methyl-D-serine, *N*-ethyl-L-serine, *N-*ethyl-DL-serine, *N*-ethyl-D-serine, *N*-methyl-L-alanine, *N*-methyl-DL-alanine, *N*-methyl-D-alanine, *N*-ethyl-L-alanine, *N*-ethyl-DL-alanine, *N*-ethyl-D-alanine, *N-*carbobenzoyloxy-L-serine, *N*-carbobenzoyloxy-DL-serine, *N*-carbobenzoyloxy-D-serine, *N*-carbobenzoyloxy-L-alanine, *N*-carbobenzoyloxy-DL-alanine, *N-*carbobenzoyloxy-D-alanine, *N*-cyclohexyl-glycine, *N*-cyclohexyl-DL-serine, *N-*cyclohexyl-L-serine, *N*-cyclohexyl-D-serine, *N*-cyclohexyl-L-alanine, *N*-cyclohexyl-DL-alanine, *N*-cyclohexyl-D-alanine, N-benzenesulfonyl-glycine, *N*-benzenesulfonyl-L-serine, *N*-benzenesulfonyl-DL-serine, *N*-benzenesulfonyl-D-serine, *N-*benzenesulfonyl-L-alanine, *N*-benzenesulfonyl-DL-alanine, and *N*-benzenesulfonyl-L-alanine. Many women on the panel were unable to continue with the irritation test due to strong sensory irritation caused by 3% β-alanine, which is disclosed as a conventional active substance.

External compositions for skin are shown below as examples of preparation examples according to the present invention. Each of these compositions had an excellent effect, such as parakeratosis inhibition, pore shrinkage, or rough skin preventing/ameliorating.

### Pharmaceutical preparation 1 Face lotion

| Component | Amount added (mass%) |
|---|---|
| (1) 1,3-Butylene glycol | 6.0 |
| (2) Glycerin | 4.0 |
| (3) Oleyl alcohol | 0.1 |
| (4) POE (20) sorbitan ester monolaurate | 0.5 |
| (5) POE (15) lauryl alcohol ester | 0.5 |
| (6) Ethanol | 10.0 |
| (7) N-benzenesulfonyl-glycine | 3.0 |
| (8) Purified water | Balance |

### (Preparation Method)

(1) and (2) were dissolved in purified water (8) at room temperature to obtain an aqueous phase. (3), (4), and (5) were dissolved in ethanol (6) and mixed with the aqueous phase to obtain a microemulsion. Next, N-benzenesulfonyl-glycine (7) was added. The product was filtered and packaged to obtain a face lotion.

### Preparation 2 through 31; Face lotion

The face lotions of preparation 2 through 31 were prepared similar to the preparation example 1 by mixing the following amounts of components in place of the 3.0 wt% of *N*-benzenesulfonyl-glycine of the components of pharmaceutical preparation 1. The amount of purified water added was adjusted so that the total amount of components added was brought to 100 mass% in each of the preparation examples: 3.0 mass% of *N*-methyl-L-serine (preparation example 2), 3.0 mass% of *N*-methyl-DL-serine (preparation example 3), 3.0 mass% of *N*-methyl-D-serine (preparation example 4), 3.0 mass% of *N*-ethyl-L-serine (preparation example 5), 3.0 mass% of *N*-ethyl-DL-serine(preparation example 6), 3.0 mass% of *N*-ethyl-D-serine (preparation example 7), 3.0 mass% of *N*-methyl-L-alanine (preparation example 8), 3.0 mass% of *N*-methyl-DL-alanine (preparation example 9), 3.0 mass% of *N*-methyl-D-alanine (preparation example 10), 3.0 mass% of *N*-ethyl-L-alanine (preparation example 11), 3.0 mass% of *N*-ethyl-DL-alanine (preparation example 12), 3.0 mass% of *N*-ethyl-D-alanine (preparation example 13), 3.0 mass% of *N*-carbobenzoyloxy-L-serine (preparation example 14), 3.0 mass% of *N*-carbobenzoyloxy-DL-serine (preparation example 15), 3.0 mass% of *N*-carbobenzoyloxy-D-serine (preparation example 16), 3.0 mass% of *N*-carbobenzoyloxy-L-alanine (preparation example 17), 3.0 mass% of *N*-carbobenzoyloxy-DL-alanine (preparation example 18), 3.0 mass% of *N*-carbobenzoyloxy-D-alanine (preparation example 19), 3.0 mass% of *N-*cyclohexyl-glycine (preparation example 20), 3.0 mass% of *N*-cyclohexyl-DL-serine (preparation example n 21), 3.0 mass% of *N*-cyclohexyl-L-serine (preparation example 22), 3.0 mass% of *N*-cyclohexyl-D-serine (preparation example 23), 3.0 mass% of *N*-cyclohexyl-L-alanine (preparation example 24), 3.0 mass% of *N-*cyclohexyl-DL-alanine (preparation example 25), 3.0 mass% of *N*-cyclohexyl-D-alanine (preparation example 26), 3.0 mass% of *N*-benzenesulfonyl-L-serine (preparation example 27), 3.0 mass% of *N*-benzenesulfonyl-DL-serine (preparation example 28), 3.0 mass% of *N*-benzenesulfonyl-D-serine (preparation example 29),3.0 mass% of *N*-benzenesulfonyl-L-alanine (preparation example 30), 3.0 mass% of *N*-benzenesulfonyl-DL-alanine (preparation example 31), and 3.0 mass% of *N*-benzenesulfonyl-L-alanine (preparation example 32).

### Preparation Example 33;; Face lotion

| Component | Amount added (mass%) |
|---|---|
| (Alcohol phase) | |
| (1) Ethanol | 10.0 |
| (2) Oleyl alcohol | 0.1 |
| (3) POE (20) sorbitan ester monolaurate | 0.5 |
| (4) POE (15) lauryl ether | 0.5 |
| (5) Preservative | As needed |
| (6) Fragrance | As needed |

| (Aqueous phase) | |
|---|---|
| (7) *N*-cyclohexyl-glycine | 3.0 |
| (8) Glycyl-glycine | 1.0 |
| (9) 1,3-Butylene glycol | 6.0 |
| (10) Glycerin | 4.0 |
| (11) Deionized water | Balance |

### (Preparation Method)

The aqueous phase and alcohol phase were each prepared and then mixed.

### Preparation Examples 34 through 65; Face Lotion

The face lotions of preparation examples 34 through 65 were prepared similary to preparation example 33 by mixing the following amounts of components in place of the 3.0 wt% of *N*-cyclohexyl-glycine of the components of preparation example 33. The amount of deionized water added was adjusted so that the total amount of components added was brought to 100 mass% in each of the preparation examples: 3.0 mass% of *N*-methyl-L-serine (preparation example 34), 3.0 mass% of *N*-methyl-DL-serine (preparation example 35), 3.0 mass% of *N*-methyl-D-serine (preparation example 36), 3.0 mass% of *N*-ethyl-L-serine (preparation example 37), 3.0 mass% of *N*-ethyl-DL-serine(preparation example 38), 3.0 mass% of *N*-ethyl-D-serine (preparation example 39), 3.0 mass% of *N*-methyl-L-alanine (preparation example 40), 3.0 mass% of *N*-methyl-DL-alanine (preparation example 41), 3.0 mass% of *N*-methyl-D-alanine (pharmaceutical preparation 42), 3.0 mass% of *N-*ethyl-L-alanine (preparation example 43), 3.0 mass% of *N*-ethyl-DL-alanine (preparation example 44), 3.0 mass% of *N*-ethyl-D-alanine (preparation example 45), 3.0 mass% of *N*-carbobenzoyloxy-L-serine (preparation example 46), 3.0 mass% of *N*-carbobenzoyloxy-DL-serine (preparation example 47), 3.0 mass% of *N-*carbobenzoyloxy-D-serine (preparation example 48), 3.0 mass% of *N-*carbobenzoyloxy-L-alanine (preparation example 49), 3.0 mass% of *N-*carbobenzoyloxy-DL-alanine (preparation example 50), 3.0 mass% of *N-*carbobenzoyoxy-D-alanine (preparation example 51), 3.0 mass% of *N*-cyclohexyl-DL-serine (preparation example 52), 3.0 mass% of *N*-cyclohexyl-L-serine (preparation example 53), 3.0 mass% of *N*-cyclohexyl-D-serine (preparation example 54), 3.0 mass% of *N*-cyclohexyl-L-alanine (preparation example 55), 3.0 mass% of *N-*cyclohexyl-DL-alanine (preparation example 56), 3.0 mass% of *N*-cyclohexyl-D-alanine (preparation example 57), 3.0 mass% of *N*-benzenesulfonyl-glycine (preparation example 58), 3.0 mass% of *N*-benzenesulfonyl-L-serine (preparation example 59), 3.0 mass% of *N*-benzenesulfonyl-DL-serine (preparation example 60), 3.0 mass% of *N*-benzenesulfonyl-D-serine (preparation example 61),3.0 mass% of *N*-benzenesulfonyl-L-alanine (preparation example 62), 3.0 mass% of *N-*benzenesulfonyl-DL-alanine (preparation example 63), 3.0 mass% of *N-*benzenesulfonyl-L-alanine (preparation example 64), and 1.0 mass% of *N-*cyclohexyl-glycine and 1.0 mass% of carbobenzoyloxy-L-serine (preparation example 65).

### Preparation example 66; Cream

| Component | Amount added (mass%) |
|---|---|
| (1) Stearyl alcohol | 6.0 |
| (2) Stearic acid | 2.0 |
| (3) Hydrogenated lanolin | 4.0 |
| (4) Squalene | 9.0 |
| (5) Octyl dodecanol | 10.0 |
| (6) 1,3-butylene glycol | 6.0 |
| (7) PEG1500 | 4.0 |
| (8) POE(25)cetyl alcohol ester | 3.0 |
| (9) Glycerin monostearate | 2.0 |
| (10) *N*-cyclohexyl-DL-alanine | 3.0 |
| (11) ACES | 1.0 |
| (12) Tocopherol | 0.1 |
| (13) Purified water | Balance |

### (Preparation Method)

(6) and (7) were added to purified water (13) and heated to 70°C. (1) through (5) were heated and dissolved, (8), (9), and (12) were added, and the mixture was brought to 70°C. (10) and (11) were then added. This mixture was added to the aqueous phase, the emulsified particles were homogenized with a homomixer, and the product was degassed, filtered, and cooled to obtain a cream.

### Preparation Examples 67 through 98; Cream

The cream of preparation examples 67 through 98 was prepared similar to preparation example 66 by mixing the following amounts of components in place of the 3.0 wt% of *N*-cyclohexyl-DL-alanine of the components of Preparation Example 66. The amount of purified water added was adjusted so that the total amount of components added was brought to 100 mass% in each of the preparation examples: 3.0 mass% of *N*-methyl-L-serine (preparation example 67), 3.0 mass% of *N*-methyl-DL-serine (preparation example 68), 3.0 mass% of *N*-methyl-D-serine (preparation example 69), 3.0 mass% of *N*-ethyl-L-serine (preparation example 70), 3.0 mass% of *N*-ethyl-DL-serine (preparation example 71), 3.0 mass% of *N*-ethyl-D-serine (preparation example 72), 3.0 mass% of *N*-methyl-L-alanine (preparation example 73), 3.0 mass% of *N*-methyl-DL-alanine (preparation example 74), 3.0 mass% of *N-*methyl-D-alanine (preparation example 75), 3.0 mass% of *N*-ethyl-L-alanine (preparation example 76), 3.0 mass% of *N*-ethyl-DL-alanine (preparation example 77), 3.0 mass% of *N*-ethyl-D-alanine (preparation example 78), 3.0 mass% of *N-*carbobenzoyloxy-L-serine (preparation example 79), 3.0 mass% of *N-*carbobenzoyloxy-DL-serine (preparation example 80), 3.0 mass% of *N-*carbobenzoyloxy-D-serine (preparation example 81), 3.0 mass% of *N-*carbobenzoyloxy-L-alanine (preparation example 82), 3.0 mass% of *N-*carbobenzoyloxy-DL-alanine (preparation example 83), 3.0 mass% of *N-*carbobenzoyloxy-D-alanine (preparation example 84), 3.0 mass% of *N*-cyclohexyl-DL-serine (preparation example 85), 3.0 mass% of *N*-cyclohexyl-L-serine (preparation example 86), 3.0 mass% of *N*-cyclohexyl-D-serine (preparation example 87), 3.0 mass% of *N*-cyclohexyl-L-alanine (preparation example 88), 3.0 mass% of *N-*cyclohexyl-D-alanine (preparation example 89), 3.0 mass% of *N*-cyclohexyl-glycine (preparation example 90), 3.0 mass% of *N*-benzenesulfonyl-glycine (preparation example 91 ),3.0 mass% of N-benzenesulfonyl-L-serine (preparation example 92), 3.0 mass% of *N*-benzenesulfonyl-DL-serine (preparation example 93), 3.0 mass% of *N*-benzenesulfonyl-D-serine (preparation example 94),3.0 mass% of *N-*benzenesulfonyl-L-alanine (preparation example 95), 3.0 mass% of *N-*benzenesulfonyl-DL-alanine (preparation example 96), 3.0 mass% of *N-*benzenesulfonyl-L-alanine (preparation example 97), and 1.0 mass% of *N-*cyclohexyl-glycine and 1.0 mass% of benzenesulfonyl-L-serine (preparation example 98).

### Preparation Example 99; Cream

| Component | Amount added (mass%) |
|---|---|
| (1) Stearic acid | 5.0 |
| (2) Stearyl alcohol | 4.0 |
| (3) Isopropyl myristate | 18.0 |
| (4) Glycerin ester monostearate | 3.0 |
| (5) Propylene glycol | 10.0 |
| (6) *N*-benzoyl-β-alanine | 1.0 |
| (7) *N*-cyclohexyl-L-serine | 2.0 |
| (8) Glycyl-glycine | 1.0 |
| (9) Potassium hydroxide | 0.2 |
| (10) Sodium bisulfite | 0.01 |
| (11) Preservative | As needed |
| (12) Fragrance | As needed |
| (13) Deionized water | Balance |

### (Preparation Method)

Propylene glycol, *N*-benzoyl-β-alanine, *N*-cyclohexyl-L-serine, glycyl-glycine, and potassium hydroxide were added to deionized water and dissolved into the remaining deionized water, heated, and maintained at 70°C (aqueous phase). The other components were mixed, heated, melted, and maintained at 70°C (oil phase) The oil phase was gradually added to the aqueous phase, pre-emulsified, uniformly emulsified using a homomixer, and cooled to 30°C while being thoroughly stirred.

### Preparation Example 100; Clarifying Lotion

| Component | Amount added (mass%) |
|---|---|
| (Phase A) | |
| (1) Ethyl alcohol (95%) | 10.0 |
| (2) POE (20) octyl dodecanol | 1.0 |
| (3) Pantothenyl ethyl ether | 0.1 |
| (4) ASDA.4Na | 1.5 |
| (5) Methyl paraben | 0.15 |
| (6) Ethanol | 10.0 |

| (Phase B) | |
|---|---|
| (7) Potassium hydroxide | 0.1 |

| (Phase C) | |
|---|---|
| (8) Glycerin | 5.0 |
| (9) Dipropylene glycol | 10.0 |
| (10) *N*-carbobenzoyloxy-L-serine | 2.0 |
| (11) Carboxyvinyl polymer | 0.2 |
| (12) Purified water | Balance |

### (Preparation method)

Phases A and C were uniformly dissolved, and phase A was added to phase C to obtain a microemulsion. Then phase B was added and mixed.

### Preparation Example 101; Milky Lotion

| Component | Amount added (mass%) |
|---|---|
| (1) Stearic acid | 2.5 |
| (2) Cetyl alcohol | 1.5 |
| (3) Vaseline | 5.0 |
| (4) Liquid paraffin | 10.0 |
| (5) POE (10) monooleate | 2.0 |
| (6) PEG 1500 | 3.0 |
| (7) Triethanolamine | 1.0 |
| (8) *N*-benzenesulfonyl-DL-serine | 0.5 |
| (9) Sodium bisulfite | 0.01 |
| (10) Ethyl paraben | 0.3 |
| (11) Carboxyvinyl polymer | 0.05 |
| (12) Fragrance | As needed |
| (13) Deionized water | Balance |

### (Preparation method)

Carboxyvinyl polymer was dissolved in a small amount of deionized water (phase A). PEG 1500, N-benzenesulfonyl-DL-serine, and triethanol amine were added and heated, dissolved, and maintained at 70°C (aqueous phase). The other components were mixed, heated, melted, and maintained at 70°C (oil phase). The oil phase was added to the aqueous phase and pre-emulsified, phase A was added and uniformly emulsified with a homomixer, and the product was cooled to 30°C while being thoroughly agitated.

### Preparation Example 102; Gel

| Component | Amount added (mass%) |
|---|---|
| (1) 95% Ethanol | 10.0 |
| (2) Dipropylene glycol | 15.0 |
| (3) POE (15) oleyl alcohol [eth]er (*8) | 2.0 |
| (4) *N*-methyl-DL-serine | 1.0 |
| (5) Sodium bisulfite | 0.03 |
| (6) Carboxyvinyl polymer ("Carbopol 941") | 1.0 |
| (7) Potassium hydroxide | 0.15 |
| (8) *L*-arginine | 0.1 |
| (9) Fragrance | As needed |
| (10) Preservative | As needed |
| (11) Purified water | Balance |

### (Preparation method)

(4) and (6) were uniformly dissolved in purified water (11) (aqueous phase). On the other hand, (2), (3), (5), (9), and (10) were dissolved in (1) and the product was added to the aqueous phase. The mixture was neutralized and thickened by (7) and (8) to obtain a gel.

### Preparation Example 103; Pack

| Component | Amount added (mass%) |
|---|---|
| (Phase A) | |
| Dipropylene glycol | 5.0 |
| POE(60)hydrogenated castor oil | 5.0 |

| (Phase B) | |
|---|---|
| Olive oil | 5.0 |
| Tocopherol acetate | 0.2 |
| Ethyl paraben | 0.2 |
| Fragrance | 0.2 |

| (Phase C) | |
|---|---|
| *N*-carbobenzoyloxy-L-alanine | 1.0 |
| Sodium bisulfite | 0.3 |
| Polyvinyl alcohol (degree of saponification of 90, degree of polymerization of 2,000) | 13.0 |
| Ethanol | 7.0 |
| Deionized water | Balance |

### (Preparation Method)

Phases A, B, and C were uniformly dissolved, and phase B was added to phase A to obtain a microemulsion. Next, the microemulsion was added to phase C and mixed.

### Preparation Example 104; Peel-off Pack

| Component | Amount added (mass%) |
|---|---|
| (Alcohol phase) | |
| 95% Ethanol | 10.0 |
| POE(15) Oleyl alcohol ether | 2.0 |
| Preservative | As needed |
| Fragrance | As needed |

| (Aqueous phase) | |
|---|---|
| *N*-ethyl-L-serine | 3.0 |
| Glutathione | 3.0 |
| Arbutin | 3.0 |
| Polyvinyl alcohol | 12.0 |
| PEG1500 | 1.0 |
| Deionized water | Balance |

### (Preparation Method)

The aqueous phase was prepared at 80°C and then cooled to 50°C. The alcohol phase prepared at room temperature was subsequently added, and then uniformly mixed and set aside to cool.

### Preparation Example; Powdered Pack

| Components | Amount added (mass%) |
|---|---|
| (Alcohol phase) | |
| 95% Ethanol | 2.0 |
| Preservative | As needed |
| Fragrance | As needed |
| Pigment | As needed |

| (Aqueous phase) | |
|---|---|
| *N*-Benzenesulfonyl-L-alanine | 3.0 |
| Propylene glycol | 7.0 |
| Zinc oxide | 25.0 |
| Kaolin | 20.0 |
| Deionized water | Balance |

### (Preparation method)

The aqueous phase was uniformly prepared at room temperature. Then the alcohol phase prepared at room temperature was added and uniformly mixed.

### Preparation Example 106; Solid Powder Foundation

| Component | Amount added (mass%) |
|---|---|
| (1) Talc | 15.0 |
| (2) Sericite | 10.0 |
| (3) Spherical nylon powder | 10.0 |
| (4) Porous silicate anhydride powder | 15.0 |
| (5) Boron nitride | 5.0 |
| (6) Titanium dioxide | 5.0 |
| (7) Iron oxide | 3.0 |
| (8) Zinc stearate | 5.0 |
| (9) *N*-cyclohexyl-DL-alanine | 3.0 |
| (10) Liquid paraffin | Balance |
| (11) Glycerin triisooctanoate | 15.0 |
| (12) Sorbitan sesquioleate | 1.5 |
| (13) Preservative | As needed |
| (14) Fragrance | As needed |

### (Preparation method)

After components (1) through (8) were mixed and ground, a mixture of components (9) through (14) was added and stirred. The mixture was molded into a pot to obtain a solid powder foundation.

### Preparation Example 107; Water-in-Oil Emulsified Foundation

| Component | Amount added (mass%) |
|---|---|
| (1) Spherical nylon | 10.0 |
| (2) Porous anhydrous silicate powder | 8.0 |
| (3) Mica titanium | 2.0 |
| (4) Silicone-treated sericite | 2.0 |
| (5) Silicone-treated mica | 12.0 |
| (6) Silicone-treated titanium dioxide | 5.0 |
| (7) Silicone-treated iron oxide | 2.0 |
| (8) Deionized water | Balance |
| (9) *N*-cyclohexyl-*N*-methyl-glycine | 3.0 |
| (10) Decamethylcyclopentane siloxane | 18.0 |
| (11) Dimethyl polysiloxane | 5.0 |
| (12) Squalene | 1.0 |
| (13) POE-modified dimethyl polysiloxane | 2.0 |
| (14) Preservative | As needed |
| (15) Fragrance | As needed |

### (Preparation Method)

Having been mixed and ground, (1) through (7) were added and dispersed in a uniformly mixed solution of components (9) through (15). (8) was added to this dispersion, emulsified, and filled into a container to obtain a water-in-oil emulsified foundation.

### INDUSTRIAL APPLICABILITY

The α-amino acid derivatives represented by general formula (1) according to the present invention, and salts thereof have the excellent function of inhibiting parakeratosis, shrinking pores, and preventing/ameliorating rough skin; therefore, they are used in cosmetics containing pharmaceutical external products, pharmaceuticals, food products, and various other fields as parakeratosis inhibitors, pore-shrinking agents, and rough skin preventing/ameliorating agents. Moreover, α-amino acid derivatives represented by general formula (1), and salts thereof are added, in particular, to an external composition for skin and used cosmetic products including non-medical products, pharmaceuticals, and other fields as an external composition for skin having a function such as parakeratosis inhibition, pore shrinkage, or rough skin prevention/abatement.

## Claims

1. A parakeratosis inhibitor agent, pore-shrinking agent, or rough skin preventing/ameliorating agent comprising one, two, or more compounds selected from the group consisting of an α-amino acid derivative represented by general formula (1), or a salt thereof. 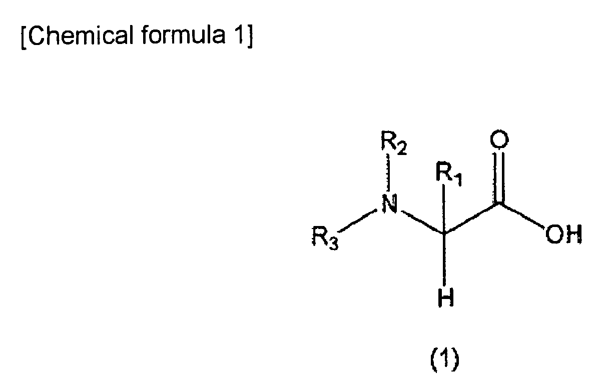 (In general formula (1), R₁ represents a hydrogen atom, a CH₃ group, or a CH₂OH group. R₂ and R₃ each independently represents a hydrogen atom, an alkyl group having 1 to 3 carbons, an allyl group, a carbobenzoyloxy group, a group represented by the following general formula (2), or a group represented by the following general formula (3). R₂ and R₃ cannot both be hydrogen groups. When R₁ is a hydrogen atom, one of R₂ and R₃ is a group represented by general formula (2) or general formula (3).) 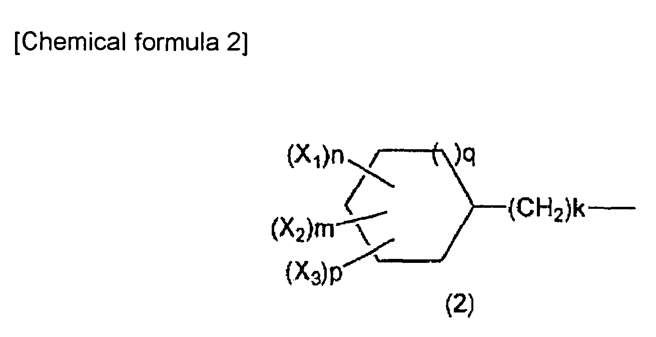 (In general formula (2), X₁, X₂, and X₃ each independently represents an alkyl group having 1 to 4 carbons, an alkoxy group having 1 to 4 carbons, a hydroxyl group, an amino group, a monoalkylamino having 1 to 4 carbons or dialkylamino group, a fluorine atom, or a trifluoromethyl group. n, m, and p each independently represents an integer of 0 to 3, and k and q each independently represents an integer of 0 to 2.) 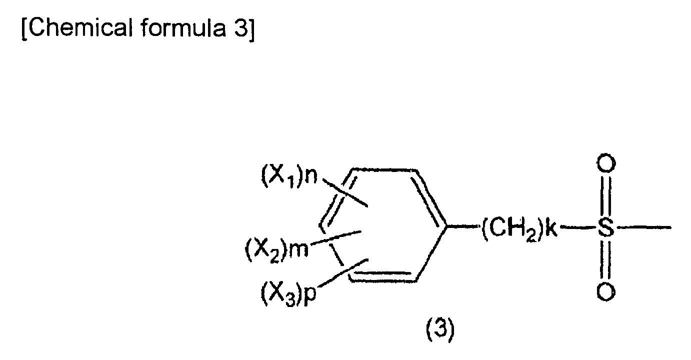 (In general formula (3), X₁, X₂, X₃, k, n, m, and p are the same similarly to general formula (2).)

2. The parakeratosis inhibitor agent, pore-shrinking agent, or rough skin preventing/ameliorating agent according to claim 1, wherein one of R₂ and R₃ in general formula (1) of claim 1 is a hydrogen atom.

3. The parakeratosis inhibitor agent, pore-shrinking agent, or rough skin preventing/ameliorating agent according to claim 1 or 2, wherein one of R₂ and R₃ in general formula (1) of claim 1 is an alkyl group having 1 to 3 carbons.

4. The parakeratosis inhibitor agent, pore-shrinking agent, or rough skin preventing/ameliorating agent according to claim 1 or 2, wherein one of R₂ and R₃ in general formula (1) of claim 1 is a carbobenzoyloxy group.

5. The parakeratosis inhibitor agent, pore-shrinking agent, or rough skin preventing/ameliorating agent according to claim 1 or 2, wherein one of R₂ and R₃ in general formula (1) of claim 1 is a cyclohexyl group.

6. The parakeratosis inhibitor agent, pore-shrinking agent, or rough skin preventing/ameliorating agent according to claim 1 or 2, wherein one of R₂ and R₃ in general formula (1) of claim 1 is a benzenesulfonyl group.

7. The parakeratosis inhibitor agent, pore-shrinking agent, or rough skin preventing/ameliorating agent according to claim 1, wherein the α-amino acid derivative represented by general formula (1) of claim 1 is *N*-methyl-L-serine, *N*-methyl-DL-serine, *N*-methyl-D-serine, *N*-ethyl-L-serine, *N*-ethyl-DL-serine, *N*-ethyl-D-serine, *N*-methyl-L-alanine, *N*-methyl-DL-alanine, *N*-methyl-D-alanine, *N*-ethyl-L-alanine, *N*-ethyl-DL-alanine, *N*-ethyl-D-alanine, *N*-carbobenzoyloxy-L-serine, *N*-carbobenzoyloxy-DL-serine, *N*-carbobenzoyloxy-D-serine, *N*-carbobenzoyloxy-L-alanine, *N*-carbobenzoyloxy-DL-alanine, *N*-carbobenzoyloxy-D-alani ne, *N*-cyclohexyl-glycine, *N*-cyclohexyl-DL-serine, *N*-cyclohexyl-L-serine, *N*-cyclohexyl-D-serine, *N*-cyclohexyl-L-alanine, *N*-cyclohexyl-DL-alanine, *N*-cyclohexyl-D-alanine, *N*-benzenesulfonyl-glycine, *N*-benzenesulfonyl-L-serine, *N*-benzenesulfonyl-DL-serine, *N*-benzenesulfonyl-D-serine, *N*-benzenesulfonyl-L-alanine, *N*-benzenesulfonyl-DL-alanine, and *N*-benzenesulfonyl-L-alanine.

8. An external composition for skin, **characterized in** comprising the parakeratosis inhibitor agent, pore-shrinking agent, or rough skin preventing/ameliorating agent according to any of claims 1 through 7.
